(19) Europäisches Patentamt
European Patent Office
Office européen des brevets

(11) Numéro de publication : **0 512 902 A1**

(12) **DEMANDE DE BREVET EUROPEEN**

(21) Numéro de dépôt : **92401236.2**

(22) Date de dépôt : **30.04.92**

(51) Int. Cl.$^5$ : **C07D 211/26**, C07D 401/06, A61K 31/445

(30) Priorité : **03.05.91 FR 9105489**

(43) Date de publication de la demande : **11.11.92 Bulletin 92/46**

(84) Etats contractants désignés : **AT BE CH DE DK ES FR GB GR IT LI LU NL PT SE**

(71) Demandeur : **ELF SANOFI**
**32-34, rue Marbeuf**
**F-75008 Paris (FR)**

(72) Inventeur : **Emonds-Alt, Xavier**
**La Balajade**
**F-34980 Combaillaux (FR)**
Inventeur : **Grossriether, Isabelle**
**Rue Abel Brunyer**
**F-30700 Uzes (FR)**
Inventeur : **Proietto, Vincenzo**
**1, Cour de Merle**
**F-34680 St Georges D'Orques (FR)**
Inventeur : **Van Broeck, Didier**
**367, avenue du Champ des Moulins**
**F-34570 Murviel Les Montpellier (FR)**

(74) Mandataire : **Gillard, Marie-Louise et al**
**Cabinet Beau de Loménie 55, Rue d'Amsterdam**
**F-75008 Paris (FR)**

(54) **Nouveaux composés dialkylènepipéridino et leurs énantiomères, procédé pour leur préparation et compositions pharmaceutiques les contenant.**

(57) La présente invention concerne des dérivés aromatiques de formule :

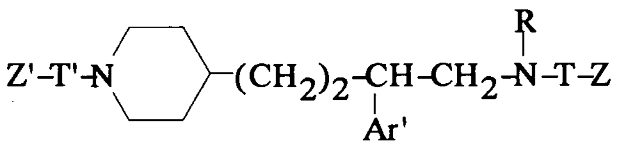

(I)

dans laquelle :

— Ar′ représente un phényle non substitué ou substitué une ou plusieurs fois par un atome d'halogène, de préférence un atome de chlore ou de fluor, par un alkyle en $C_1$-$C_3$, par un trifluorométhyle, par un alcoxy en $C_1$-$C_3$, par un hydroxyle ; un groupe thiényle, pyridyle, naphtyle, lesdits groupes étant non substitués ou substitués par un halogène de préférence un atome de chlore ou de fluor, un groupe indolyle ou un groupe benzothiényle ;

— R représente l'hydrogène, un groupe méthyle ou un groupe $(CH_2)_n$-L, où n est un nombre entier de 2 à 6 et L est l'hydrogène ou un groupe amino ;

— Z et Z′ représentent indépendamment un atome d'hydrogène ou un groupe M ou OM,
M représente l'hydrogène, un alkyle droit ou ramifié en $C_1$-$C_6$ ; un $\alpha$-hydroxybenzyle, un $\alpha$-alkylbenzyle ou un phénylalkyle dans lequel le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone ; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (1-méthyl) 2-imidazolylthioalkyle dans lequel le groupe alkyle comprend de 1 à 3 atomes de carbone ; un 1-oxo-3-phénylindan-2-yle ; un groupe aromatique ou hétéroaromatique ledit groupe étant non substitué ou substitué ;

— T′ représente une liaison, un groupe $-CH_2-$ ou, un groupe -C(O)- ;

— T représente un groupe choisi parmi

EP 0 512 902 A1

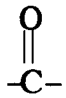 et 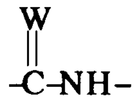

W étant un atome d'oxygène ou de soufre, avec la limitation que

. lorsque Z′ est hydrogène ou OM, T′ est autre qu'une liaison ; et

. lorsque Z est hydrogène ou OM, T est autre qu'un groupe -C(W)-NH ;

ou un de leurs sels éventuels avec des acides minéraux ou organiques, ou leurs sels d'ammonium quaternaire.

<u>APPLICATION</u> : traitement de toute pathologie substance P et neurokinine dépendante, notamment l'analgésie et l'inflammation.

La présente invention a pour objet de nouveaux dérivés aromatiques substitués par un groupement amino et par diverses fonctions esters, amines ou amides, ainsi que leurs énantiomères.

La présente invention concerne également le procédé d'obtention des composés, qui peut être énantiosélectif et l'utilisation des composés selon l'invention dans des compositions à usage thérapeutique et plus particulièrement dans les phénomènes pathologiques qui impliquent le système des neurokinines comme : la douleur (D. Regoli et al., Life Sciences, 1987, 40, 109-117), l'allergie et l'inflammation (J.E. Morlay et al., Life Sciences, 1987, 41, 527-544), l'insuffisance circulatoire (J. Losay et al., 1977, Substance P, Von Euler, U.S. and Pemow ed., 287-293, Raven Press, New York), les troubles gastro-intestinaux (D. Regoli et al., Trends Pharmacol. Sci., 1985, 6, 481-484), les troubles respiratoires (J. Mizrahi et al., Pharmacology, 1982, 25, 39-50).

Des ligands endogènes aux récepteurs des neurokinines ont été décrits, telles la substance P (SP), la neurokinine A (NKA) (S.J. Bailey et al., 1983, Substance P, P. Skrabanck ed., 16-17 Boole Press, Dublin) et la neurokinine B (NKB) (S.P. Watson, Life Sciences, 1983, 25, 797-808).

Les récepteurs aux neurokinines ont été reconnus sur de nombreuses préparations et sont actuellement classés en trois types : $NK_1$, $NK_2$ et $NK_3$. Alors que la plupart des préparations étudiées jusqu'à maintenant, présentent plusieurs types de récepteurs, tel l'iléon de cobaye ($NK_1$, $NK_2$ et $NK_3$), certaines d'entre elles n'en possèderaient qu'un seul, telles l'artère carotide de chien ($NK_1$), l'artère pulmonaire de lapin dépourvue d'endothélium ($NK_2$) et la veine porte de rat ($NK_3$) (D. Regoli et al., Trends Pharmacol. Sci., 1988, 9, 290-295 et Pharmacology, 1989, 38, 1-15).

Une caractérisation plus précise des différents récepteurs est rendue possible par la synthèse récente d'agonistes sélectifs. Ainsi, la [$Sar^9$, Met-$(O_2)^{11}$] SP, la [$Nle^{10}$] $NKA_{4-10}$, et la [Me $Phe^7$] -NKB présenteraient une sélectivité respective pour les récepteurs $NK_1$, $NK_2$ et $NK_3$ (cf D. Regoli, 1988 et 1989 précédemment cité).

On a maintenant trouvé que certains composés aromatiques aminés possèdent des propriétés pharmacologiques intéressantes, en interagissant avec les récepteurs des neurokinines et sont notamment utiles pour le traitement de toute pathologie substance P et neurokinine dépendante, notamment l'analgésie et l'inflammation.

Ainsi selon un de ses aspects, la présente invention concerne des dérivés aromatiques aminés de formule :

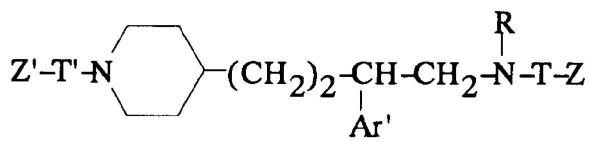

(I)

dans laquelle :

- Ar' représente un phényle non substitué ou substitué une ou plusieurs fois par un atome d'halogène, de préférence un atome de chlore ou de fluor, par un alkyle en $C_1$-$C_3$, par un trifluorométhyle, par un alcoxy en $C_1$-$C_3$, par un hydroxyle ; un groupe thiényle, pyridyle, naphtyle, lesdits groupes étant non substitués ou substitués par un halogène de préférence un atome de chlore ou de fluor, un groupe indolyle ou un groupe benzothiényle ;
- R représente l'hydrogène, un groupe méthyle ou un groupe $(CH_2)_n$-L, où n est un nombre entier de 2 à 6 et L est l'hydrogène ou un groupe amino ;
- Z et Z' représentent indépendamment un atome d'hydrogène ou un groupe M ou OM,

M représente l'hydrogène, un alkyle droit ou ramifié en $C_1$-$C_6$ ; un α-hydroxybenzyle, un α-alkylbenzyle ou un phénylalkyle dans lesquels le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone ; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (1-méthyl) 2-imidazolylthioalkyle dans lequel le groupe alkyle comprend de 1 à 3 atomes de carbone ; un 1-oxo-3-phénylindan-2-yle ; un groupe aromatique ou hétéroaromatique ledit groupe étant non substitué ou substitué ;

- T' représente une liaison, un groupe $-CH_2-$ ou, un groupe -C(O)-;
- T représente un groupe choisi parmi

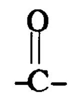

et

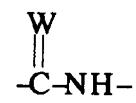

W étant un atome d'oxygène ou de soufre, avec la limitation que

. lorsque Z′ est hydrogène ou OM, T′ est autre qu'une liaison ; et

. lorsque Z est hydrogène ou OM, T est autre qu'un groupe -C(W)-NH;

ou un de leurs sels éventuels avec des acides minéraux ou organiques, ou leurs sels d'ammonium quaternaire.

Les sels d'ammonium quarternaire des composés de formule (I) sont formés à partir de l'atome d'azote de la pipéridine.

Le groupe

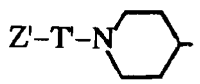

est alors représenté par le groupe:

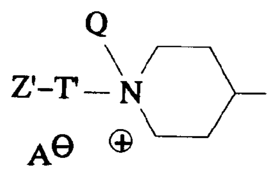

dans lequel

. Q représente un groupe alkyle en $C_1$-$C_6$ ou un groupe benzyle et

. $A^{\ominus}$ représente un anion choisi parmi chlorure, bromure, iodure, acétate, méthanesulfonate ou paratoluènesulfonate.

Dans la présente description, les groupes alkyle ou les groupes alcoxy sont droits ou ramifiés.

Les sels des composés de formule (I) selon la présente invention comprennent aussi bien ceux avec des acides minéraux ou organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate, le glycolate, le gluconate, le citrate, l'iséthionate.

De façon particulière, dans la formule (I), Z et/ou Z′ représentent un groupe aromatique ou hétéroaromatique mono-, di- ou tricyclique, pouvant porter un ou plusieurs substituants, dont un atome de carbone du carbocycle aromatique ou de l'hétérocycle aromatique est directement lié au groupe T ou au groupe T′.

Plus particulièrement, les radicaux Z et/ou Z peuvent être un groupe phényle ou benzyle, qui peuvent être non substitués ou éventuellement contenir un ou plusieurs substituants.

Lorsque Z et/ou Z′ sont un groupe phényle ou benzyle, ceux-ci peuvent être de préférence mono- ou disubstitué notamment en position 2,4, mais aussi par exemple en position 2,3, 4,5, 3,4 ou 3,5 ; il peut aussi être trisubstitué, notamment en position 2,4,6 mais aussi par exemple en 2,3,4, 2,3,5 ou 2,4,5 3,4,5 ; tétrasubstitué, par exemple en 2,3,4,5 ou pentasubstitué. Les substituants des groupes phényle ou benzyle peuvent être F; Cl; Br; I; CN; OH; $NH_2$; NH-CO-$NH_2$; $N0_2$; $CONH_2$ ; $CF_3$ ; alkyle en $C_1$-$C_{10}$, de préférence en $C_1$-$C_4$, méthyle ou éthyle étant préférés, ainsi que par exemple n-propyle, isopropyle, n-butyle, isobutyle, sec-butyle, tert.-butyle, pentyle ou n-pentyle, hexyle ou n-hexyle, heptyle ou n-heptyle, octyle ou n-octyle, nonyle ou n-nonyle ainsi que décyle ou n-décyle ; alcényle contenant 2 à 10, de préférence 2-4 atomes de carbone, par exemple vinyle, allyle, 1-propényle, isopropényle, butényle ou 1-buten-1-, -2-, -3- ou -4-yle, 2-buten-1-yle, 2-buten-2-yle, pentényle, hexényle ou décényle ; alcynyle contenant 2 à 10, de préférence 2-4 atomes de carbone, par exemple éthynyle, 1-propyn-1-yle, propargyle, butynyle ou 2-butyn-1-yle, pentynyle, décynyle ; cycloalkyle contenant 3 à 8, de préférence 5 ou 6 atomes de carbone, cyclopentyle ou cyclohexyle étant préférés, ainsi que par exemple cyclopropyle, cyclobutyle, 1-, 2- ou 3-méthylcyclopentyle, 1-, 2-, 3- ou 4-méthylcyclohexyle, cycloheptyle ou cyclooctyle ; bicycloalkyle contenant 4 à 11, de préférence 7 atomes de carbone, exo ou endo 2-norbornyle étant préférés, ainsi que par exemple 2-isobornyle ou 5-camphyle ; hydroxyalkyle contenant 1 à 5, de préférence 1-2 atomes de carbone, hydroxyméthyle et 1- ou 2-hydroxyéthyle étant préférés, ainsi que par exemple 1-hydroxyprop-1-yle, 2-hydroxyprop-1-yle, 3-hydroxyprop-1-yle, 1-hydroxyprop -2-yle, 1-hydroxybut-1-yle, 1-hydroxypent-1-yle ; alcoxy contenant 1 à 10, de préférence 1-4 atomes de carbone, méthoxy ou éthoxy étant préférés, ainsi que par exemple n-propoxy, isopropoxy, n-butoxy, isobutoxy, sec-butoxy, tert-butoxy, pentyloxy, hexyloxy, heptyloxy, octyloxy, nonyloxy ou décyloxy ; alcoxyalkyle contenant 2 à 10, de préférence de 2 à 6 atomes de carbone, par exemple alcoxyméthyle ou alcoxyéthyle, tel que méthoxyméthyle ou 1- ou 2-méthoxyéthyle, 1- ou 2-n-butoxyéthyle, 1- ou 2-n-octyloxyéthyle ; alcoxyalcoxyalkyle contenant de 3 à 10, de préférence de 4 à 7 atomes de carbone, par exemple alcoxyalcoxyméthyle,par exemple 2-méthoxyéthoxyméthyle, 2-éthoxyéthoxyméthyle ou 2-isopropoxyéthoxyméthyle, alcoxyalcoxyéthyle par exemple 2-(2-méthoxyéthoxy)éthyle ou 2-(2-éthoxyéthoxy)éthyle ; alcoxyalcoxy contenant de 2 à 10, de préférence de 3 à 6 atomes de carbone, par exemple 2-méthoxyéthoxy, 2-éthoxyéthoxy ou 2-n-butoxyéthoxy ; alcényloxy contenant 2 à 10, de préférence 2 à 4 atomes de carbone, allyloxy étant préféré, ainsi que par exemple vinyloxy, propényloxy, isopropényloxy, butényloxy tel que 1-butèn-1-, -2-, -3- ou -4-yloxy, 2-butèn-1-yloxy, 2-butèn-2-yloxy, pentènyloxy, hexényloxy ou décényloxy ; alcényloxyalkyle contenant de 3 à 10, de préférence 3-6 atomes de carbone, par exemple allyloxyméthyle ; alcynyloxy contenant de 2 à 10, de préférence 2 à 4 atomes de carbone, propargyloxy étant préféré, ainsi que par exemple éthynyloxy, 1-propyn-1-yloxy, butynyloxy ou 2-butyn-1-yloxy, pentynyloxy ou décynyloxy ; alcynyloxyalkyle contenant de 3 à 10 de préférence 3 à 6 atomes de carbone, par exemple éthylnyloxyméthyle, propargyloxyméthyle ou 2-(2-butyn-1-yloxy)éthyle ; cycloalcoxy contenant 3 à 8, de préférence 5 ou 6 atomes de carbone, cyclopentyloxy ou cyclohexyloxy étant préférés, ainsi que par exemple cyclopropyloxy, cyclobutyloxy, 1-, 2- ou 3-méthylcyclopentyloxy, 1-, 2-, 3- ou 4-méthylcyclohexyloxy, cycloheptyloxy ou cyclooctyloxy ; alkylthio contenant de 1 à 10, de préférence 1 à 4 atomes de carbone, méthylthio ou éthylthio étant préférés, ainsi que par exemple n-propylthio, isopropylthio, n-butylthio, isobutylthio, sec-butylthio, tert-butylthio, pentylthio, hexylthio, octylthio, nonylthio ou décylthio ; alkylthioalkyle contenant de 2 à 10, de préférence 2 à 6 atomes de carbone, par exemple méthylthiométhyle, 2-méthylthioéthyle ou 2-n-butylthioéthyle acylamino, à savoir alcanoylamino contenant de 1 à 7, de préférence 1 à 4 atomes de carbone, formylamino et acétylamino étant préférés, ainsi que propionylamino, butyrylamino, isobutyrylamino, valérylamino, caproylamino, heptanoylamino, ainsi que aroylamino ou benzoylamino ; acylaminoalkyle, de préférence alcanoylaminoalkyle contenant de 2 à 8, de préférence 3 à 6 atomes de carbone, tel que formylaminoéthyle, acétylaminoéthyle, propionylaminoéthyle, n-butyrylaminoéthyle, formylaminopropyle, acétylaminopropyle, propionylaminopropyle, formylaminobutyle, acétylaminobutyle, ainsi que propionylaminobutyle, butyrylaminobutyle ; acyloxy contenant de 1 à 6, de préférence 2 à 4 atomes de carbone, acétyloxy, propionyloxy ou butyryloxy étant préférés, ainsi que par exemple formyloxy, valéryloxy, caproyloxy ; alcoxycarbonyle contenant de 2 à 5, de préférence 2 ou 3 atomes de carbone, méthoxycarbonyle et éthoxycabonyle étant préférés, ainsi que par exemple n-propoxycarbonyle, isopropoxycarbonyle, n-butoxycarbonyle, isobutoxycarbonyle, sec-butoxycarbonyle ou tert-butoxycarbonyle ; cycloalcoxycarbonyle contenant de 4 à 8, de préférence 6 ou 7 atomes de carbone, cyclopentyloxycarbonyle, cyclohexyloxycarbonyle étant préférés, ainsi que cyclopropyloxycarbonyle, cyclobutyloxycarbonyle ou cycloheptyloxycarbonyle ; alkylaminocarbonylamino contenant de 2 à 4 atomes de carbone, tel que méthylaminocarbonylamino, éthylaminocarbonylamino, propylaminocarbonylamino ; dialkylaminocarbonylamino contenant de 3 à 7, de préférence 3 à 5 atomes de carbone, de préférence diméthylaminocarbonylamino, ainsi que di-n-propylaminocarbonylamino, diisopropylaminocarbonylamino ; (1-pyrrolidino)-carbonylamino; (1-pipéridino)-carbonylamino; cycloalkylaminocarbonylamino contenant de 4 à 8, de préférence 6 ou 7 atomes de carbone, cyclopentylaminocarbonylamino, cyclohexylaminocarbonylamino étant préférés, ainsi que cyclopropylaminocarbonylamino, cyclobutylaminocarbonylamino, cycloheptylaminocarbonylamino; alkylaminocarbonylaminoalkyle contenant de 3 à 9, de préférence 4 à 7 atomes de carbone, méthylaminocarbonylaminoéthyle, éthylaminocarbonylaminoéthyle, éthylaminocarbonylaminopropyle, éthylaminocarbonylaminobutyle étant préférés, ainsi que par exemple méthylaminocarbonylaminométhyle, n-propylaminocarbonylaminobutyle, n-butylaminocarbonylaminobutyle dialkylaminocarbonylaminoalkyle contenant de 4 à 11 atomes de carbone, par exemple diméthylaminocarbonylaminométhyle, diéthylaminocarbonylaminoéthyle, diéthylaminocarbonyl-aminopropyle, diéthylaminocarbonylaminobutyle, (1-pyrrolidino) carbonylaminoéthyle, (1-pipéridino)- carbonylaminoéthyle; cycloalkylaminocarbonylaminoalkyle contenant de 5 à 12, de préférence 8 à 11 atomes de carbone, cyclopentylaminocarbonylaminoéthyle, cyclopentylaminocarbonylaminopropyle, cyclopentylaminocarbonylaminobutyle, cyclohexylaminocarbonylaminoéthyle, cyclohexylaminocarbonylaminopropyle et cyclohexylaminocarbonylaminobutyle étant préférés, ainsi que par exemple cyclopropylaminocarbonylaminoéthyle, cycloheptylaminocarbonylaminoéthyle ; alcoxycarbonylaminoalkyle contenant de 3 à 12, de préférence 4 à 9 atomes de carbone, méthoxycarbonylaminoéthyle, éthoxycarbonylaminoéthyle,

n-propoxycarbonyleaminoéthyle, isopropoxycarbonylaminoéthyle, n-butoxycarbonylaminoéthyle, isobutoxycarbonylaminoéthyle, sec-butoxycarbonylaminoéthyle, tert-butoxycarbonylaminoéthyle, éthoxycarbonylaminopropyle, n-butoxycarbonylaminopropyle, éthoxycarbonylaminobutyle, n-butoxycarbonylaminobutyle étant préférés, ainsi que par exemple n-propoxycarbonylaminopropyle, n-propoxycarbonylaminobutyle, isopropoxycarbonylaminobutyle ; cycloalcoxycarbonylaminoalkyle contenant de 5 à 12, de préférence 8 à 11 atomes de carbone, cyclopentyloxycarbonylaminoéthyle, cyclopentyloxycarbonylaminopropyle, cyclopentyloxycarbonylaminobutyle, cyclohexyloxycarbonylaminoéthyle, cyclohexyloxycarbonylaminopropyle, cyclohexyloxycarbonylaminobutyle étant préférés, ainsi que par exemple cyclopropyloxycarbonylaminométhyle, cycloheptyloxycarbonylaminoéthyle carbamoylalkyle contenant de 2 à 5, de préférence 2 atomes de carbone, de préférence carbamoylméthyle, ainsi que carbamoyléthyle, carbamoylpropyle, carbamoylbutyle ; alkylaminocarbonylalkyle contenant de 3 à 9, de préférence 3 à 6 atomes de carbone, méthylaminocarbonyléthyle, éthylaminocarbonylméthyle, n-propylaminocarbonylméthyle, isopropylaminocarbonylméthyle, n-butylaminocarbonylméthyle, isobutylaminocarbonylméthyle, sec-butylaminocarbonylméthyle, tert-butylaminocarbonylméthyle étant préférés, ainsi que par exemple éthylaminocarbonyléthyle, éthylaminocarbonylpropyle, éthylaminocarbonylbutyle, n-propylaminocarbonylbutyle, n-butylaminocarbonylbutyle; dialkylaminocarbonylalkyle contenant de 4 à 11, de préférence 4 à 8 atomes de carbone, diméthylaminocarbonylméthyle,diéthylaminocarbonylméthyle, di-n-propylaminocarbonylméthyle ainsi que par exemple diéthylaminocarbonyléthyle, diéthylaminocarbonylpropyle, diéthylaminocarbonylbutyle; (1-pyrrolidino)carbonylméthyle, (1- pipéridino) carbonyl-méthyle ; (1-pipéridino)carbonyléthyle ; cycloalkylaminocarbonylalkyle contenant de 5 à 12, de préférence 7 ou 8 atomes de carbone, cyclopentylaminocarbonylméthyle et cyclohexylaminocarbonylméthyle étant préférés, ainsi que par exemple cyclopropylaminocarbonylméthyle, cyclobutylaminocarbonylméthyle, cycloheptylaminocarbonylméthyle, cyclohexylaminocarbonyléthyle, cyclohexylaminocarbonylpropyle, cyclohexylaminocarbonylbutyle ; al kylaminocarbonylalcoxy contenant de 3 à 10, de préférence 3 à 5 atomes de carbone, méthylaminocarbonylméthoxy étant préféré, ainsi que par exemple méthylaminocarbonyléthoxy, méthylaminocarbonylpropoxy ; dialkylaminocarbonylalcoxy contenant de 4 à 10, de préférence 4 à 7 atomes de carbone, tel que diméthylaminocarbonylméthoxy, diéthylaminocarbonyléthoxy, (pipéridinyl-1) carbonylméthoxy ; cycloalkylaminocarbonylalcoxy contenant de 5 à 11, de préférence 7 ou 8 atomes de carbone, tel que cyclopentylaminocarbonylméthoxy, cyclohexylaminocarbonylméthoxy.

Les groupes Z et Z′ sont avantageusement un groupe phényle ; un groupe benzyle ; un groupe benzoyle; un groupe phénylthioalkyle dans lequel l'alkyle est en $C_1$-$C_3$; un groupe naphtyle.

Le groupe phényle Z ou Z′ est de préférence mono ou disubstitué par un halogène,ou par un alcoxy en $C_1$-$C_4$, le groupe 2,4-dichlorophényle étant particulièrement préféré.

Les radicaux Z ou Z′ peuvent également représenter un groupe aromatique bicyclique tels que le 1- ou 2-naphtyle ; 1-, 2-, 3-, 4-, 5-, 6-, 7-indényle ; dont une ou plusieurs liaisons peuvent être hydrogénées, lesdits groupes pouvant être non substitués ou contenir éventuellement un ou plusieurs substituants tel que : un halogène et plus particulièrement un atome de fluor, le groupe alkyle, phényle, cyano, hydroxyalkyle, hydroxy, oxo, alkylcarbonylamino et alcoxycarbonyle, thioalkyle dans lesquels les alkyles sont en $C_1$-$C_4$.

Les radicaux Z ou Z′ peuvent être aussi un groupe pyridyle, thiadiazolyle, indolyle, indazolyle, imidazolyle, benzimidazolyle, quinolyle, benzotriazolyle, benzofurannyle, benzothiényle, benzothiazolyle, benzisothiazolyle, isoquinolyle, benzoxazolyle, benzisoxazolyle, benzoxazinyle, benzodioxinyle ou pyridinyle, isoxazolyle, benzopyranyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, alkylcarbonylamino et alcoxycarbonyle, thioalkyle dans lesquels les alkyles sont en $C_1$-$C_4$.

Selon un autre de ses aspects, la présente invention concerne un procédé pour la préparation de composés aromatiques aminés différemment substitués de formule (I) et de leurs sels.

Dans un premier temps, on prépare le dérivé N-protégé et O-protégé du 1-hydroxy-2-(4-pipéridinyl)éthane selon les méthodes classiques et habituelles en utilisant les groupes protecteurs bien connus de l'homme de l'art pour les groupes N-protecteurs ou pour les groupes O-protecteurs, selon le schéma 1 suivant, pour obtenir l'aminoalcool protégé qui est un intermédiaire nouveau faisant partie de l'invention.

## SCHEMA 1

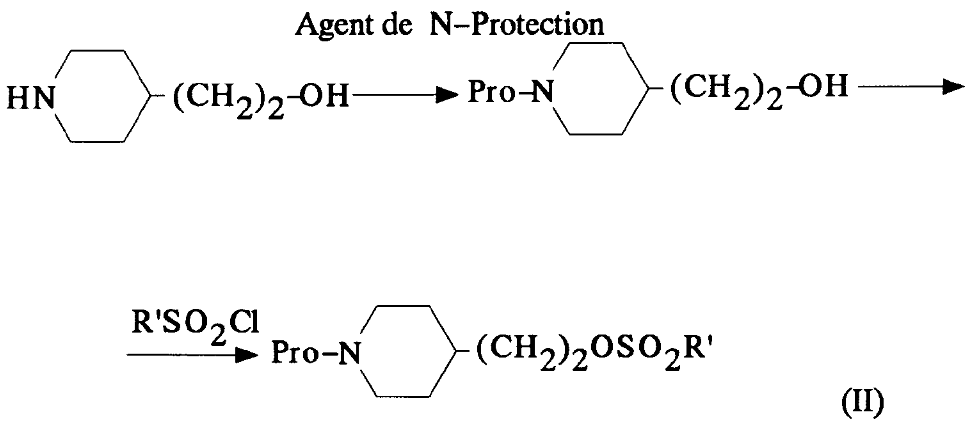


(II)

dans lequel:

- Pro désigne un groupe N-protecteur,
- R′ représente un groupe alkyle en $C_1$-$C_4$, un groupe phényle ou un groupe tolyle, de préférence paratolyle.

Le composé de formule (II) est préparé à partir du 2-(4-pipéridinyl-)-éthanol, produit disponible commercialement.

Dans la présente description on utilise les termes "groupe N-protecteur", "N-protégé" et "N-déprotection" ou tout simplement "déprotection".

Le terme "groupe N-protecteur" indique un groupe protecteur du groupe amino du type de ceux utilisés dans la chimie des peptides ou des nucléotides, par exemple un groupe acyle, comme formyle, acétyle, propionyle ; un groupe alcoxycarbonyle, comme t.butoxycarbonyle (Boc) ; un groupe alcoxyalkylcarbonyle, comme méthoxypropionyle; un groupe alcoxycarbonyle substitué, comme trichloroéthoxycarbonyle un groupe alkylcarbonyle substitué, comme monochlorométhylcarbonyle, monochloroéthylcarbonyle, dichloroéthylcarbonyle, trichlorométhylcarbonyle, trichloropropylcarbonyle; un groupe aralkyloxycarbonyle, comme benzyloxycarbonyle ; un groupe aralkyloxycarbonyle substitué, comme 4-nitrobenzyloxycarbonyle ou aroyle comme benzoyle, 2,4-dichlorobenzoyle ou 4-fluoro-1-naphthylcarbonyle. Le terme "N-protecteur" inclut également des groupes aralkyliques comme par exemple un groupe benzyle, non substitué ou substitué par exemple avec 1 ou 2 atomes d'halogène, de préférence le chlore ou avec 1 ou 2 groupes alcoxy, de préférence méthoxy ; un groupe diphénylméthyle (ou benzhydryle), un groupe diphénylméthyle substitué comme di(4-méthoxy) diphénylméthyle (ou diméthoxybenzhydryle), un groupe triphénylméthyle (ou trityle), un groupe triphénylméthyle substitué, comme 4-méthoxyphényldiphénylméthyle (ou méthoxytrityle) ou di(4-méthoxyphényl) phénylméthyle (ou diméthoxytrityle). Le terme "N-protégé" se rapporte au produit qui contient l'amine protégée par un groupe N-protecteur tel que défini ci-dessus.

Le terme "N-déprotection" ou tout simplement "déprotection" indique l'élimination du groupe N-protecteur et l'obtention de l'amine libre selon les méthodes classiques bien connues de l'homme du métier, par exemple par réduction ou par hydrolyse acide selon le groupe N-protecteur à éliminer.

Le procédé de préparation des composés (I) est illustré par le Schéma 2.

Dans ce Schéma, les composés de formules VII et I sont indiqués comme directement obtenus des composés IV (voie (b)), XII (voie (a)) et IV (voie (c)). En réalité, lorsque R est un substituant autre que l'hydrogène, ledit substituant est introduit sur l'amine des composés XII (voie (a)), IV (voie (b)) et IV (voie (c)) selon les méthodes décrites en détail ci-dessous. La même remarque vaut pour les Schémas 3 et 4.

SCHEMA 2

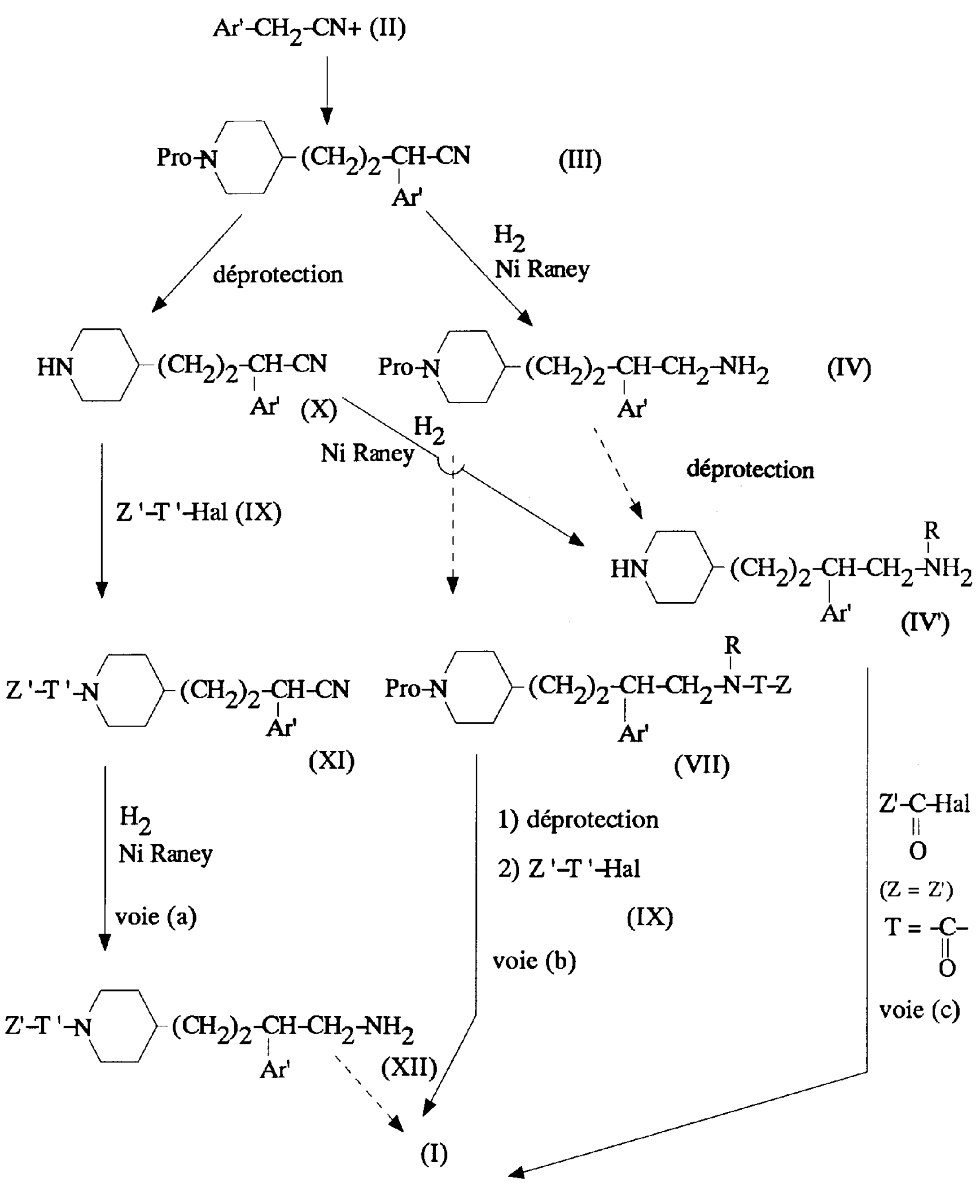


Les composés de départ de formule (III) sont préparés à partir des nitriles de formule :

$$Ar'-CH_2-CN$$

dans lequel Ar' est tel que défini précédemment, nitriles qui sont des produits commerciaux ou préparés selon des méthodes connues, par réaction avec un composé de formule (II) ; puis l'aminonitrile N-protégé de

formule (III) est traité selon l'une des voies de synthèse (a), (b) ou (c), lesquelles utilisent les mêmes réactions mais dans des ordres différents.

Ainsi selon la voie (b), on soumet le nitrile III à une hydrogénation catalytique en présence d'un catalyseur tel que par exemple le nickel de Raney pour fournir l'amine de formule :

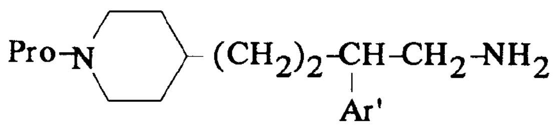

(IV)

qui est un intermédiaire nouveau faisant partie de l'invention et qui est ensuite fonctionnalisée

. soit avec un dérivé fonctionnel d'un acide de formule :

HO - CO - Z (V)

dans laquelle Z est tel que défini précédemment, lorsqu'on doit préparer un composé de formule (I) dans lequel T est - CO -,

. soit avec un iso(thio)cyanate de formule :

W = C = N - Z (VI)

dans laquelle W et Z sont tels que définis précédemment lorsqu'on soit préparer un composé de formule (I) dans lequel T est - C (W) - NH -, pour fournir le dérivé de formule :

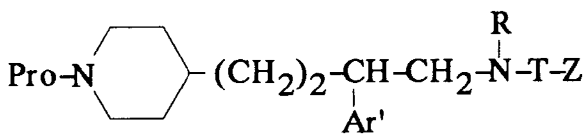

(VII, R = H)

qui est un intermédiaire nouveau faisant partie de l'invention et qui est alors déprotégé pour conduire à l'amine libre de formule :

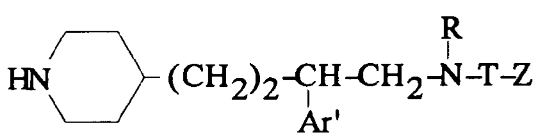

(VIII, R = H)

dans laquelle Ar', T et Z sont tels que définis prédédemment, puis cette amine est additionnée sur un dérivé halogéné de formule :

Z' - T' - Hal (IX)

dans laquelle Z' et T' sont tels que définis précédemment et où Hal représente un halogène, plus particulièrement un atome de chlore ou de brome, pour conduire aux composés (I) selon l'invention.

Selon la voie de synthèse (a) l'aminonitrile (III) est déprotégé selon les méthodes habituelles pour conduire à l'aminonitrile libre de formule :

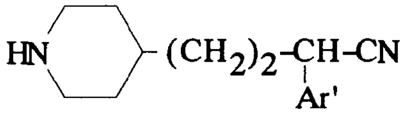

(X)

dans laquelle Ar′ est tel que défini précédemment, et sur lequel on additionne le dérivé halogéné (IX) pour conduire au nitrile de formule :

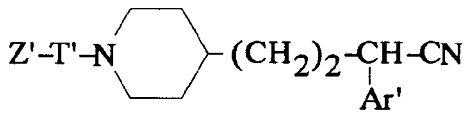

(XI)

dans laquelle Z′, T, Ar′ sont tels que définis précédemment. Ce composé (XI) est ensuite hydrogéné en présence d'un catalyseur tel que le nickel de Raney pour fournir le dérivé aminé correspondant de formule :

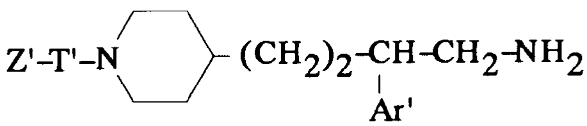

(XII)

qui est un intermédiaire nouveau faisant partie de l'invention et qui est ensuite fonctionnalisé, pour fournir le composé (I) selon l'invention en procédant de la même façon que précédemment pour la préparation des composés (I) à partir des intermédiaires (VII), c'est à dire en le faisant réagir :

. soit avec un dérivé fonctionnel d'un acide de formule :

HO - CO - Z (V)

dans laquelle Z est tel que défini précédemment, lorsqu'on doit préparer un composé de formule (I) dans lequel T est - CO -,

. soit avec un iso(thio)cyanate de formule :

W = C = N - Z (VI)

dans laquelle W et Z sont tels que définis précédemment lorsqu'on doit préparer un composé de formule (I) dans lequel T est - C (W) - NH -, pour fournir les dérivés (I) où R = H.

Comme dérivé fonctionnel de l'acide (V), on utilise l'acide lui-même, convenablement activé par exemple par le cyclohexylcarbodiimide ou par l'hexafluorophosphate de benzotriazolyl N-oxytrisdiméthylaminophosphonium (BOP), ou bien un des dérivés fonctionnels qui réagissent avec les amines, par exemple un anhydride, un anhydride mixte, le chlorure ou un ester activé. Lorsque Z est un groupe OM, l'acide concerné est l'acide carbonique et, comme dérivé fonctionnel, on utilise le monochlorure, à savoir un chloroformiate Cl-CO-OM.

Selon la voie de synthèse (c), on soumet le nitrile (III) à une hydrogénation comme dans la voie (b), puis on déprotège le composé (IV) obtenu pour former le composé (IV′) correspondant, que l'on fait réagir ensuite avec le dérivé halogéné Z′-C(O) - Hal pour former le composé de formule (I) dans lequel Z = Z′.

En variante, le composé de formule (IV) peut être obtenu à partir du composé de formule (X) par hydrogénation en présence d'un catalyseur, tel que le nickel de Raney.

La préparation des composés de formule (VII) ou (I), où R est autre que l'hydrogène est effectuée selon des méthodes connues en soi.

Lorsqu'on souhaite préparer les composés de formule (IV) ou (XII) dans lesquels R est méthyle, on traite l'amine libre, obtenue par hydrogénation des nitriles (III) ou (XI) comme décrit ci-dessus, avec un chloroformiate, par exemple avec le chloroformiate de formule Cl - CO - OAlk, où Alk est un alkyle en $C_1$-$C_3$, de préférence éthyle, pour obtenir les carbamates de formule :

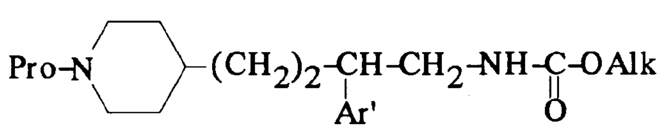

(XIV)

ou

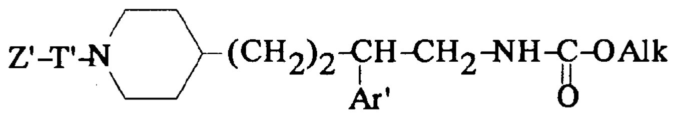

(XV)

qui sont ensuite réduits par des moyens connus, tel que l'action d'un agent réducteur comme par exemple un hydrure métallique, tel que l'hydrure de sodium et d'aluminium, l'hydrure de lithium et d'aluminium ou par un hydrure de bore, tel que le diméthylsulfure de borane. La réduction est réalisée dans un solvant, tel que l'éther ou le toluène à une température comprise entre la température ambiante et 60°C. Les méthylamines ainsi obtenues de formule :

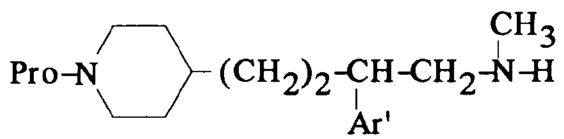

(XVI)

ou

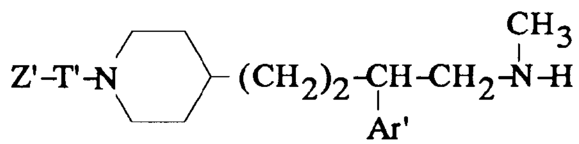

(XVII)

sont isolées selon les méthodes habituelles.

La méthylamine de formule (XVI) ci-dessus peut être déprotégée pour donner la pipéridine de formule :

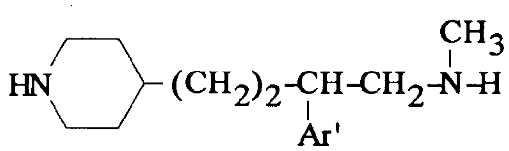

(XVI')

A partir des composés de formule (XVI), (XVI') et (XVII), on prépare les composés N-substitués de la même manière que décrite dans les schémas 2 à 4 à partir, respectivement, des composés de formule (IV) (voie (b)), (IV') (voie (c)) et (XII) (voie (a)).

Pour préparer les composés de formule (IV) ou (XII) dans lesquels R est un groupe $-(CH_2)_n-L$, où n et L sont tels que définis ci-dessus, on traite l'amine libre, obtenue par hydrogénation du nitrile (III) ou (XI) comme décrit ci-dessus, avec un dérivé fonctionnel réactif, tel que défini ci-dessus, de l'acide de formule :

$$L^\bullet-(CH_2)_{n-1} - COOH$$

dans laquelle L• est l'hydrogène ou un groupe amino protégé pour obtenir les amides de formule :

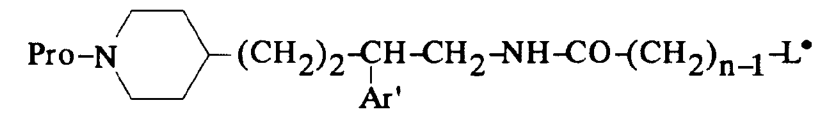

(XVIII)

ou

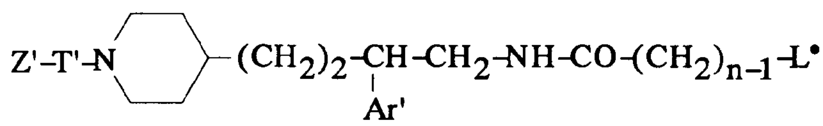

(XIX)

Les amides (XVIII) ou (XIX), par réduction dans les mêmes conditions que celles décrites ci-dessus pour les nitriles (III) ou (XI), donnent le composé désiré de formule :

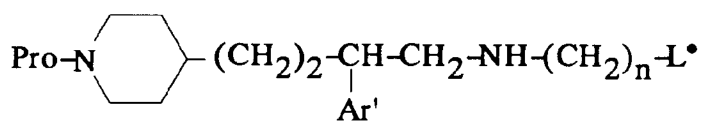

(XX)

ou

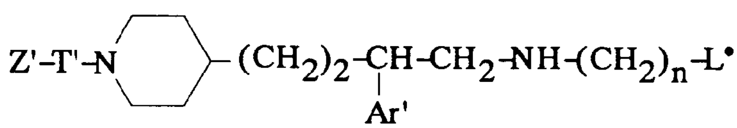

(XXI)

Les alkylamines de formule (XX) ci-dessus peuvent être partiellement déprotégées pour donner la pipéridine de formule :

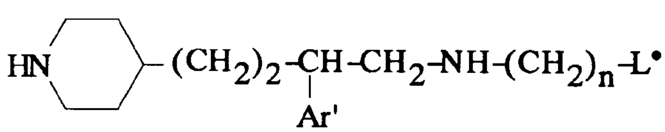

(XX')

A partir des composés de formule (XX), (XX') et (XXI) ci-dessus, on prépare les composés N-substitués de la même manière que décrit plus haut dans les schémas 2 à 4, à partir, respectivement, des composés (IV) (voie (b)), (IV') (voie (c)) et (XII) (voie (a)).

Les groupes N-protecteurs éventuellement présents dans le groupe R, lorsque L est un groupe amino, sont les groupes N-protecteurs classiques bien connus de l'homme de l'art et de préférence ceux qui sont éliminables par hydrolyse acide, tels que le groupe trityle, méthoxytrityle ou BOC.

La préparation des composés (VII) ou (I) est illustrée par les schémas 3 et 4 détaillés ci-dessous.

SCHEMA 3

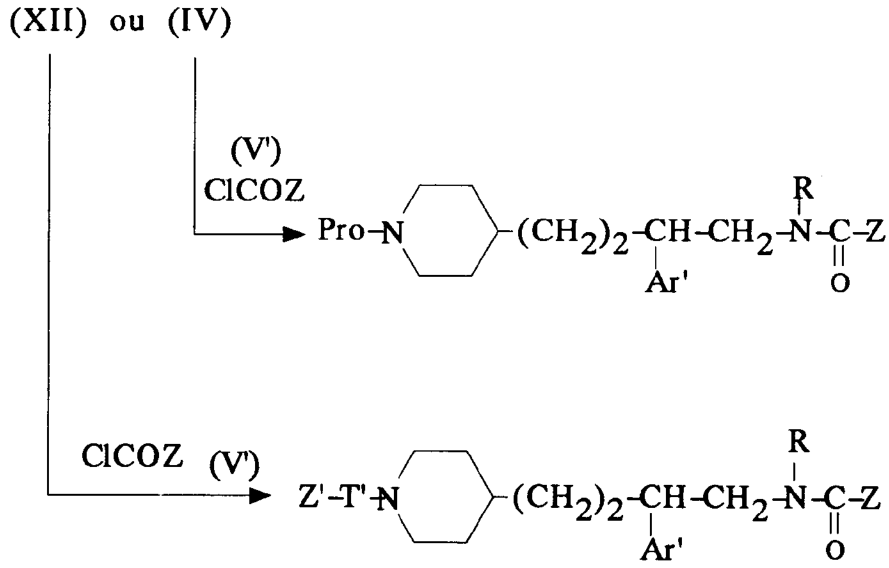


(VII1)

(I1)

SCHEMA 4

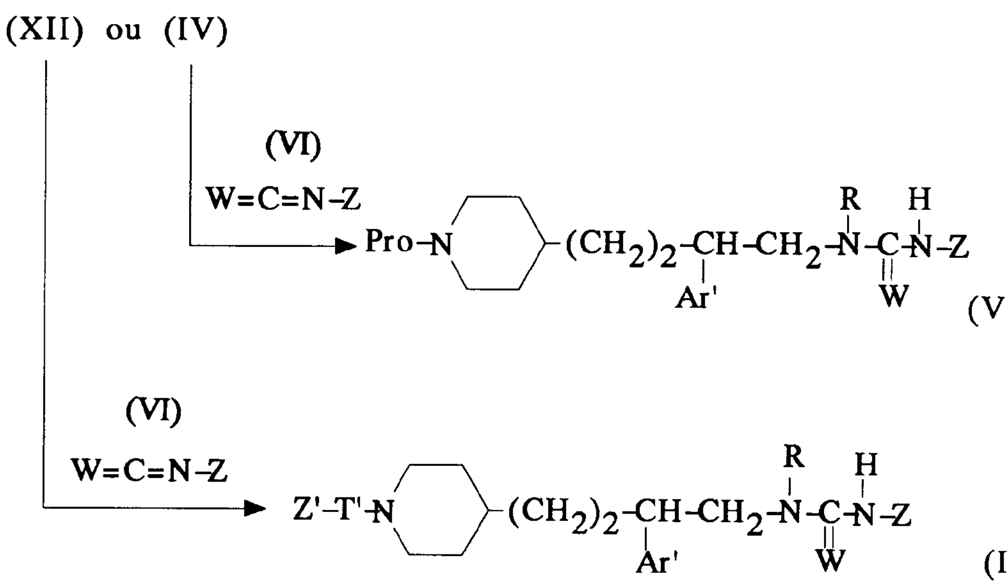


(VII2)

(I2)

On considère donc le chlorure d'acide Cl - CO - Z comme dérivé fontionnnel réactif de l'acide (V). La réaction avec le chlorure d'acide est effectuée dans un solvant inerte, tel que le dichlorométhane ou le benzène en présence d'une base telle que par exemple la triéthylamine, à température ambiante.

Dans le cas particulier de Z = OM, la réaction des composés (IV) ou (XII) avec le chloroformiate de formule :

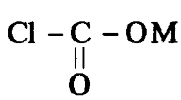

est effectuée selon les méthodes habituelles.

Lorsque Z est autre que OM, on peut utiliser un autre dérivé fonctionnel ou on peut partir de l'acide libre (V) en réalisant un couplage de (IV) ou (XII) avec le BOP (hexafluorophosphate de benzotriazolyl N-oxytrisdiméthylaminophosphonium), puis en additionnant l'acide (V) en présence d'une base organique comme par exemple la triéthylamine, dans un solvant comme le dichlorométhane ou le diméthylformamide, à température ambiante, les composés ($I_1$) obtenus étant isolés et purifiés selon les méthodes habituelles, comme par exemple la chromatographie ou la recristallisation.

On peut faire aussi réagir (IV) ou (XII) avec un iso(thio)cyanate W = C = N - Z (VI) dans un solvant inerte anhydre tel que par exemple le benzène, pendant une nuit à température ambiante puis traiter le mélange réactionnel selon les méthodes habituelles pour obtenir les composés ($I_2$).

Les produits de formule (I) ainsi obtenus sont isolés, sous forme de base libre ou de sel, selon les techniques classiques.

Lorsque le composé de formule (I) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques classiques. Ainsi, on prépare par exemple le chlorhydrate, le bromhydrate, le sulfate, l'hydrogénosulfate, le dihydrogénophosphate, le méthanesulfonate, l'oxalate, le maléate, le fumarate, la 2-naphtalènesulfonate.

A la fin de la réaction, les composés de formule (I) peuvent être isolés sous forme d'un de leurs sels, par exemple le chlorhydrate ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les sels d'ammonium quaternaire formés avec l'azote de la pipéridine sont préparés par réaction des bases libres des composés (I), pour lesquelles les fonctions aminés autres, éventuellement présentes sont N-protégées par un groupe N-protecteur habituel, avec un excès d'agent alkylant de formule :

A - Q

dans lequel A représente un groupe partant et est tel que défini précédemment pour (I), de préférence un chlorure ou un iodure et Q est tel que défini précédemment pour (I) et on chauffe le mélange réactionnel dans un solvant par exemple choisi parmi le dichlorométhane, le chloroforme, l'acétone ou l'acétonitrile à une température comprise entre la température ambiante et la température de reflux du solvant pendant une à plusieurs heures pour obtenir après traitement selon les méthodes habituelles et après déprotection éventuelle, un mélange des conformères axiaux et équatoriaux des sels d'ammonium quaternaires.

De préférence, $A^{\ominus}$ représente un iodure qui peut être échangé par un autre anion ou par un anion pharmacologiquement acceptable, par exemple un chlorure, par élution du composé (I) sur une résine échangeuse d'ion, par exemple l'Amberlite IRA68 ® ou Duolite A375 ®.

Les conformères sont séparés selon les méthodes habituelles, par exemple par chromatographie ou par recristallisation.

Chacun des conformères axiaux ou équatoriaux des composés (I) sous forme de racémiques ou sous forme d'énantiomères R ou S optiquement purs font partie de l'invention.

La résolution des mélanges racémiques (I) selon les méthodes habituelles permet d'isoler les énantiomères qui font partie de l'invention.

L'invention a également pour objet les intermédiaires mis en oeuvre dans le procédé décrit ci-dessus pour l'obtention des composés de l'invention (voir schéma 2).

Ces intermédiaires sont les arylalkylpipéridines de formule :

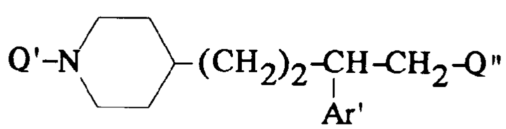

(XXII)

dans laquelle :

- Ar' est tel que défini précédemment ; et
  . soit Q' est l'hydrogène ou un groupe N-protecteur et Q" est groupe amino ou un groupe :

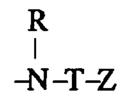

où R, T et Z sont tels que définis précédemment,

. soit Q′ est un groupe :

Z′ - T′ -

où Z′et T′ sont tels que définis précédemment et

Q″ est un groupe amino

et les sels d'addition acides desdites aralkylpipéridines lorsqu'elles sont basiques.

Les intermédiaires de synthèse de formule :

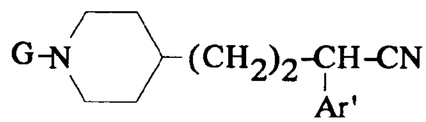

(XXIII)

dans laquelle G est l'hydrogène, un groupe protecteur (Pro) ou un groupe de formule

Z′ - T′ -

dans lequel Z′ et T′ sont tels que définis précédemment, Ar′ a la signification donnée pour les composés de formule (I), sont également des composés nouveaux qui font partie du cadre de l'invention.

Tous les intermédiaires de synthèse mis en oeuvre dans le procédé de l'invention peuvent être isolés, selon des techniques classiques bien connues de l'homme de métier, sous la forme de leurs sels.

Les composés selon l'invention ont fait l'objet d'essais biochimiques.

Les composés (I) et leurs sels ont montré des propriétés de liaison spécifique au récepteur de la substance P dans des essais réalisés sur des membranes de cortex de rat et de cellules lymphoblastiques IM9, selon M.A. Cascieri et al., J. Biol. Chem., 1983, 258, 5158-5164 et D.D. Paya et al., J. Immunol., 1984, 133, 3260-3265.

Les composés selon l'invention inhibent la fixation de la substance P à son récepteur.

Ainsi, par exemple, les composés 3, 7 et 11 inhibent la liaison de la substance P avec des Ki respectivement de 23,15 et 30 nanomolaire.

Les composés de la présente invention sont très peu toxiques. Leur toxicité est compatible avec leur utilisation en tant que principes actifs de médicament. Ces médicaments sont de préférence indiqués pour le traitement de la douleur et de l'inflammation.

Les composés de la présente invention sont généralement administrés en unité de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables.

Les composés de formule (I) ci-dessus et leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration orale, sublinguale, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration par inhalation on utilise un aérosol contenant par exemple du trioléate de sorbitane ou de l'acide oléique ainsi que du trichlorofluorométhane, du dichlorodifluorométhane, du dichlorotétrafluoroéthane ou tout autre gaz propulseur biologiquement compatible.

Le principe actif peut être formulé également sous forme de microcapsules, éventuellement avec un ou plusieurs supports ou additifs.

Les compositions susdites peuvent également renfermer d'autres produits actifs tels que, par exemple, des bronchodilatateurs, des antitussifs ou des antihistaminiques.

Les exemples suivants illustrent l'invention sans toutefois la limiter.

Les points de fusion ou de décomposition, F, ont été mesurés au banc chauffant Koffler. Les spectres de résonnance magnétique nucléaire du $^{13}C$ ont été effectués à 50 MHz dans le diméthylsulfoxyde.

EXEMPLE 1

Chlorhydrate de N-[{4-[1-benzyl-piperidin-4-yl]-2-(3,4-dichlorophenyl)}-butyl]-4-fluoronaphtalènecarboxamide(composé 1).

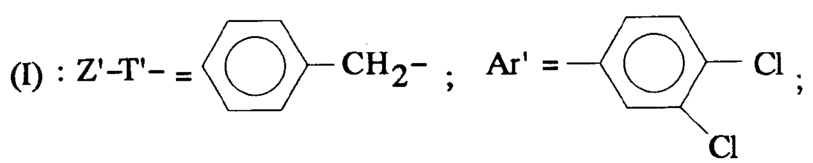

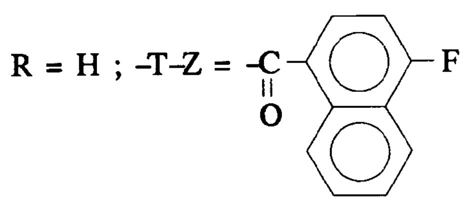

Ce composé 1 est préparé par la voie (a) du schéma 2.

A) Préparation du 4-(2-mésyloxy-1-éthynyl)-1-*tert*-butoxycarbonyl- pipéridine.

On dissout 65 g de 1-hydroxy-2-(pipéridin-4-yl)éthane dans un mélange de 250 ml de dioxane et 60 ml d'eau. On ajoute goutte à goutte 120 g de dicarbonate de di-*tert*-butyle. L'addition maintient la température du mélange réactionnel à 50-60°C. L'addition terminée, on chauffe le mélange réactionnel à 80°C puis concentre le solvant sous vide. Le résidu est repris dans l'éther et lavé successivement trois fois à l'eau puis avec une

solution saturée de NACl. La phase éthérée est séparée par décantation, séchée sur $Na_2SO_4$ et concentrée.

On obtient 110,2 g d'une huile jaunâtre.

52,5 g de l'huile préparée ci-dessus et 26,6 g de triéthylamine sont dissous dans 300 ml de dichlorométhane. La solution est refroidie dans la glace puis on additionne goutte à goutte 28,32 g de chlorure de mésyle en solution dans 5 ml de dichlorométhane. L'addition terminée, on chauffe le mélange réactionnel à reflux pendant 2 heures.

Le solvant est concentré sous vide, le résidu est repris dans l'acétate d'éthyle puis on lave successivement à l'eau, avec une solution saturée en NACl, sépare la phase organique qui est séchée sur $Na_2SO_4$ et concentrée sous vide.

Le résidu est repris dans un mélange de 70 ml d'acétate d'éthyle et 140 ml d'hexane. Les cristaux sont séparés par filtration.

m= 64g

F = 91°C.

B) Chlorhydrate de 1-(3,4-dichlorophényl)-3-(pipéridin-4-yl)-1-cyanopropane.

1,74 g d'hydrure de sodium à 55 % en suspension dans l'huile sont mis en suspension dans 150 ml de tétrahydrofuranne et refroidis à 5°C. On ajoute goutte à goutte 11,16 g de 3,4-dichlorophénylacétonitrile et 12,28 g de l'amine préparée précédemment selon A) en solution dans 150 ml de tétrahydrofuranne. Le mélange réactionnel est agité pendant une nuit à température ambiante puis chauffé à reflux pendant 30 minutes. Le solvant est concentré sous vide, le résidu est repris dans une solution tampon pH = 2 et extrait à l'éther. La phase éthérée est lavée successivement à l'eau, avec une solution saturée de NACl, séchée sur $Na_2SO_4$ et concentrée sous vide. On obtient 20,2 g de 1-(3,4-dichlorophényl)-3-[(1-t-butoxycarbonyl)-pipéridin-4-yl]-1-cyanopropane sous forme d'une huile.

On déprotège ce composé en le mettant en solution dans 100 ml d'acide trifluoroacétique et en laissant sous agitation pendant 30 minutes à température ambiante. L'acide est concentré sous vide, on obtient une huile qui est reprise dans une solution d'hydroxyde de sodium à 5 %, on extrait à l'éther, lave successivement à l'eau puis avec une solution saturée de NACl, sèche sur $Na_2SO_4$ et concentre sous vide. L'huile obtenue est dissoute dans 300 ml d'acétate d'éthyle puis on fait barboter de l'acide chlorhydrique jusqu'à coloration jaune.

Le chlorhydrate est séparé par filtration.

m = 12,4 g

F = 182°C.

C) Chlorhydrate de 3-[(1-benzyl)-pipéridin-4-yl)-1-(3,4-dichlorophényl)-1-cyanopropane.

16,75 g de l'amine préparée précédemment selon B) et 15,15 g de triéthylamine sont mis en solution dans 150 ml de dichlorométhane. On ajoute goutte à goutte 8,98 g de bromure de benzyle en solution dans 25 ml de dichlorométhane puis on chauffe à reflux pendant une heure. Le solvant est concentré sous vide puis le résidu est repris dans une solution d'hydroxyde de sodium à 5 %. On extrait à l'éther puis lave successivement à l'eau et avec une solution saturée de NACl, sèche sur $Na_2SO_4$ et concentre sous vide.

L'huile obtenue est dissoute dans 150 ml d'éthanol, on fait barboter de l'acide chlorhydrique et filtre le chlorhydrate.

m= 15 g

F = 233°C.

D) 4-[(1-Benzyl)-pipéridin-4-yl]-2-(3,4-dichlorophényl)-butylamine.

On hydrogène à pression atmosphérique et température ambiante13,3 g du produit précédemment préparé en solution dans un mélange de 150 ml d'éthanol, 20 ml d'ammoniaque concentré et en présence de 2 g de nickel de Raney. L'hydrogénation terminée, on filtre le mélange réactionnel sur célite et concentre le filtrat sous vide après avoir ajouté 2 fois 100 ml d'éthanol absolu.

On obtient 13,5 g d'une huile incolore.

E) Composé 1.

1,95 g d'amine préparée précédemment selon D), 0,95 g d'acide 4-fluoronaphtoïque et 1,01 g de triéthylamine sont mis en solution dans 40 ml de dichlorométhane. On ajoute 2,21 g de BOP (hexafluorophosphate de benzotriazolyl N-oxytrisdiméthylaminophosphonium) et laisse le mélange réactionnel à température ambiante pendant 24 heures. Le solvant est concentré sous vide, le résidu est repris dans l'éther et on lave successivement avec une solution d'hydroxyde de sodium à 5 % puis avec une solution saturée de NACl. La phase éthérée est séchée sur $Na_2SO_4$ et concentrée sous vide. Le résidu est chromatographié sur gel de silice, éluant : dichlorométhane/éthanol 93/7 (v/v).

La concentration des fractions pures fournit un résidu à partir duquel on prépare le chlorhydrate dans l'acétate d'éthyle.

m = 1,31g
F = 174-176°C.

EXEMPLE 2

Chlorhydrate de N-[{2-(3,4-dichlorophényl)-4-[1-(4-fluorobenzyl)-pipéridin-4-yl]}-butyl]-2,4-dichlorobenzamide.

(I) : Z'-T'- = 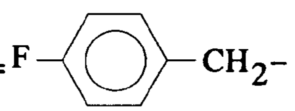 ; Ar' = 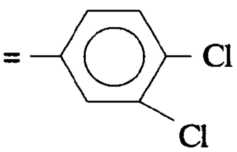 ;

R = H ; -T-Z = 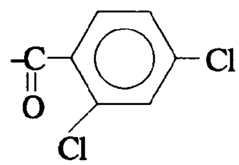

Ce composé est préparé par la voie (b) du schéma 2.

A) 1-(3,4-Dichlorophényl)-3-[(1-*tert*-butoxycarbonyl)-pipéridin-4-yl]-1-cyanopropane.

Ce composé est préparé selon l'exemple 1, étape B mais n'est pas déprotégé.

B) 2-(3,4-Dichlorophényl)-4-[(1-t-butoxycarbonyl)-pipéridin-4-yl]butylamine.

17,4 g du dérivé cyané préparé précédemment sont mis en solution dans 300 ml d'éthanol 95°, 20 ml d'eau et 70 ml d'ammoniaque concentré en présence de 2 g de nickel de Raney. On hydrogène alors à température ambiante et pression atmosphérique. L'hydrogénation terminée, on filtre le mélange réactionnel sur célite et concentre le filtrat sous vide. Le résidu est repris dans l'acétone et le précipité formé est séparé par filtration puis dissous dans l'éther, lavé avec une solution d'hydroxyde de sodium à 10 % puis avec une solution saturée de NACl. La phase éthérée est séchée sur $MgSO_4$ et concentrée sous vide.

m=16,2 g.

C) N-[{2-(3,4-Dichlorophényl)-4-[(1-*tert*-butoxycarbonyl)-pipéridin-4-yl]}-butyl]-2,4-dichlorobenzamide.

16,2 g de l'amine préparée précédemment et 8,16 g de triéthylamine sont mis en solution dans 200 ml de dichlorométhane. On ajoute alors goutte à goutte une solution de 9,31 g de chlorure de l'acide 2,4-dichlorobenzoïque, laisse le mélange réactionnel pendant trois heures à température ambiante puis chauffe à 60°C pendant 4 heures et élimine le solvant sous vide. Le résidu est repris dans l'eau et extrait à l'acétate d'éthyle. La phase organique est lavée successivement avec une solution d'hydroxyde de sodium à 10 %, deux fois à l'eau puis avec une solution saturée de NaCl, séchée avec $Na_2SO_4$ et concentrée sous vide. Le résidu est chromatographié sur gel de silice, éluant : dichlorométhane/méthanol 98/2 (v/v). La concentration des fractions pures fournit un résidu qui est utilisé tel quel pour la déprotection.

D) N-[{2-(3,4-Dichlorophényl-)-4-(pipéridin-4-yl)-}-butyl]-2,4-dichlorobenzamide.

Le résidu obtenu précédemment est repris dans une solution d'hydroxyde de sodium puis extrait au dichlorométhane. La phase organique est lavée avec une solution de NACl saturée puis séchée sur $Na_2SO_4$ et concentrée sous vide. Le résidu est cristallisé dans l'acétate d'éthyle.

m = 11,6 g

F = 118°C.

E) Composé 2

1 g du composé préparé précédemment et 0,81 g de $K_2CO_3$ sont chauffés à 60°C dans 15 ml de diméthylformamide. On ajoute alors 0,37 g de 4-fluorobromure de benzyle et continue à chauffer à 60°C sous agitation pendant une heure. Le solvant est éliminé sous vide, le résidu est repris dans l'eau puis extrait à l'acétate d'éthyle. La phase organique est lavée successivement avec une solution d'hydroxyde de sodium à 10 %, à l'eau puis avec une solution saturée de NACl, séchée sur $MgSO_4$ et concentrée sous vide.

Le résidu est chromatographié sur gel de silice, éluant : dichlorométhane/méthanol 98/2 (v/v).

La concentration des fractions pures fournit un résidu qui est repris dans l'acétate d'éthyle et dont on prépare le chlorhydrate par barbotage d'acide chlorhydrique.

m = 0,6 g

F = 110°C.

Les composés décrits dans les tableaux suivants 1, 2, 3 et 4 sont préparés selon les exemples 1 ou 2 précédents.

TABLEAU 1

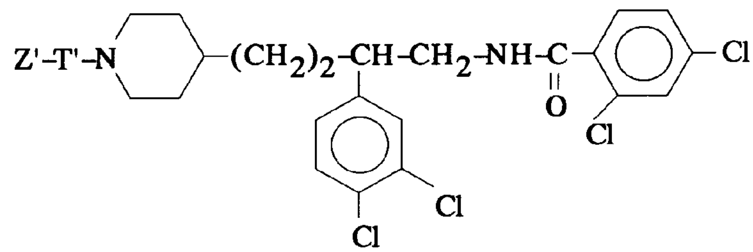

| Exemple n° | Z' | T' | F ; °C | Sel | Voie |
|---|---|---|---|---|---|
| 3 | 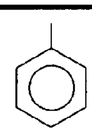 | $-CH_2-$ | 116 | HCl | a |
| 4 | 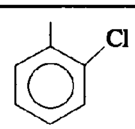 | $-CH_2-$ | 130 | HCl | b |
| 5 | 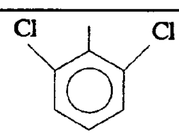 | $-CH_2-$ | 114 | HCl | b |

| TABLEAU 1 (suite) | | | | | |
|---|---|---|---|---|---|
| Exemple n° | Z' | T' | F ; °C | Sel | Voie |
| 6 | 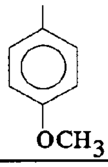 | $-CH_2-$ | 114 | HCl | b |
| 7 | 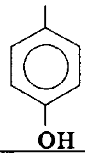 | $-CH_2-$ | 124 | HCl,$H_2O$ | b |
| 8 | 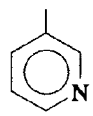 | $-CH_2-$ | 128 | HCl | b |
| 9 | 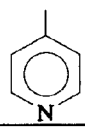 | $-CH_2-$ | 146 | 2HCl,0,5$H_2O$ | b |
| 10 | 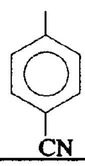 | - | 87 | 0,5$H_2O$ | b |
| 11 | 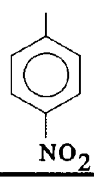 | - | 82 | base | b |

## TABLEAU 2

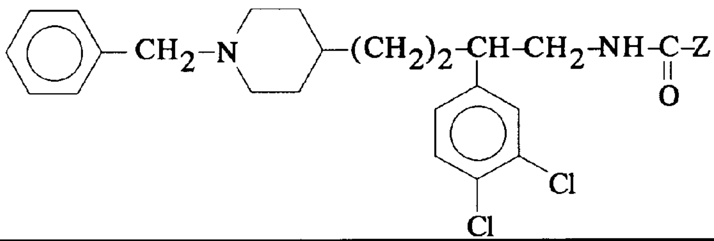

| Exemple n° | Z | F ; °C | Sel | Voie |
|---|---|---|---|---|
| 12 | 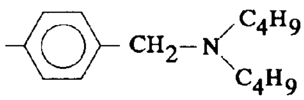 | 146 | $2HCl, 0,8H_2O$ | a |
| 13 | 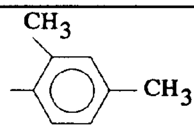 | 179 | HCl | a |

## TABLEAU 3

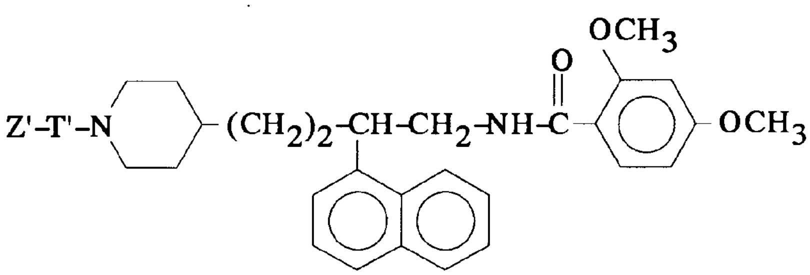

| Exemple n° | Z' | T' | F; °C | Sel | Voie |
|---|---|---|---|---|---|
| 14 | 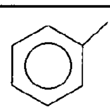 | $-CH_2-$ | 125 | HCl | a |
| 15 | 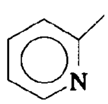 | 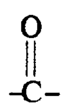 | 115 | HCl | a |

TABLEAU 4

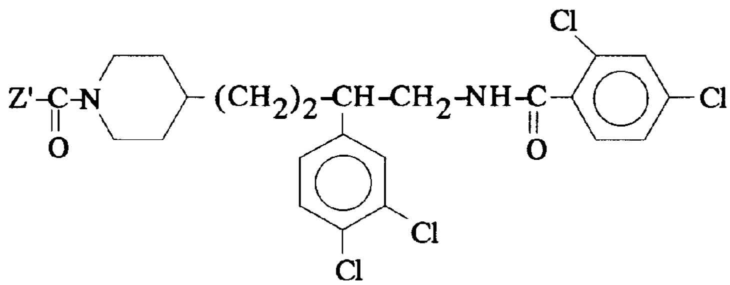

| exemple | Z' | F ; °C | Sel | Voie |
|---|---|---|---|---|
| 16 | 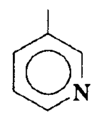 | 105 | HCl | b |
| 17 | 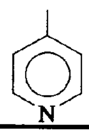 | 134 | HCl | b |

EXEMPLE 18

Chlorhydrate de N-méthyl-N-[{4-[1-benzyl-piperidin-4-yl]-2-(3,4-dichlorophenyl)}-butyl]-3-isopropoxypheny-lacétamide.

(I) : Z'-T'- = 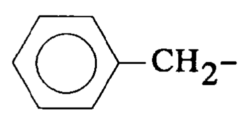 ; Ar' = 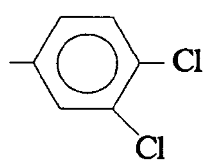 ;

R = $-CH_3$ ; -T-Z = 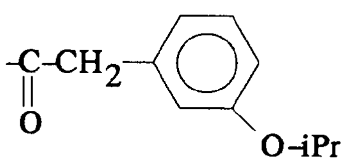

A) N-[{4-[(1-benzyl)-pipéridin-4-yl]-2-(3,4-dichlorophényl)}-butyl]carbamate d'éthyle.

2,13 g de l'amine préparé selon l'exemple 1, D) sont dissous dans 10 ml de dichlorométhane et traitées à -15°C, sous atmosphère d'azote, par 0,6 ml de chloroformiate d'éthyle et 1 ml de triéthylamine. Le mélange réactionnel est ramené à température ambiante, lavé successivement avec NAOH 5 %, $H_2O$, NACl saturé et séché sur $MgSO_4$. On obtient 2,25 d'huile incolore.

B) Chlorhydrate de N-methyl-4-[1-benzyl-piperidin-4-yl]-2-(3,4-dichlorophenyl)-butylamine.

Le carbamate obtenu précédemment est dissous dans 20 ml de THF. On ajoute le mélange à 0,50 g de $LiAlH_4$ en suspension dans 20 ml de THF. On laisse réagir 3 heures à reflux, ajoute ensuite à 0°C, 2,5 ml d'$H_2O$, filtre et évapore le filtrat. L'huile est redissoute dans du dichlorométhane et on précipité le chlorhydrate en ajoutant une solutio de HCl/éther 4N.

m = 2,45 g

F = 185°C.

C) Composé 18

1 g de la diamine précédemment préparée est mis en solution dans 15 ml de $CH_2Cl_2$. On ajoute successivement 1 ml de triéthylamine, 400 ml d'acide 3-isopropoxyphénylacétique et 1,06 g de BOP (hexafluorophosphate de benzothiazolyl N-oxytrisdiméthylaminophosphonium) et laisse le mélange à température ambiante pendant 30 minutes. Le solvant est évaporé sous vide, le résidu est repris dans l'acétate d'éthyle et lavé successivement avec NAOH 5 % et une solution d'eau saturée de NACl, séché sur $MgSO_4$ et évaporé. Le résidu est chromatographié sur gel de silice, éluant : $CH_2Cl_2/CH_3OH$ 98/2 (v/v). La concentration des fractions pures fournit un résidu à partir duquel on prépare le chlorhydrate dans le dichlorométhane.

m = 0,60 g

F = 106°C.

En procédant selon l'exemple 18 ci-dessus, on prépare les composés 19 à 22 décrits dans le tableau 5 ci-dessous.

## TABLEAU 5

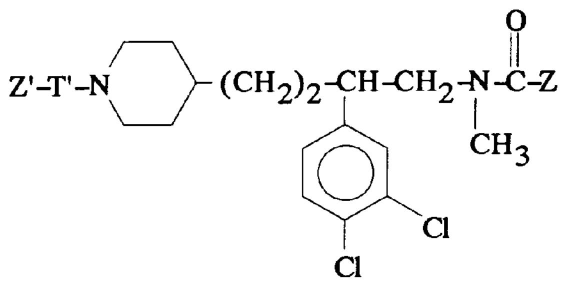

| Exemple n° | Z' | T' | F ; °C | Sel | Z | Voie |
|---|---|---|---|---|---|---|
| 19 | 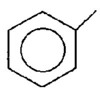 | $-CH_2-$ | 110 | HCl | 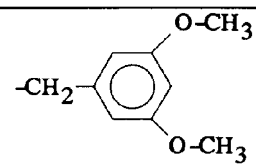 | b |
| 20 | 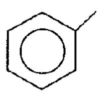 | $-CH_2-$ | 119 | HCl | 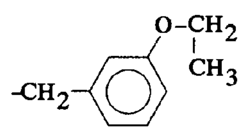 | b |
| 21 | 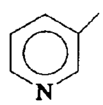 | 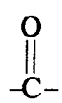 | 90 | HCl | 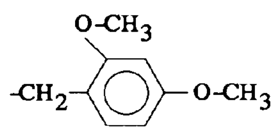 | b |
| 22 | 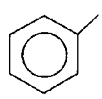 | $-CH_2-$ | 105 | HCl | 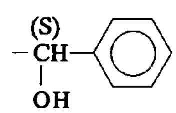 | b |

EXEMPLE 23

Chlorhydrate de N-méthyl-N-[{4-[1-4(-methyoxybenzyl)-piperidin-4-yl]-2-(3,4-dichlorophenyl)}-butyl]-3-chlorophenylacétamide.

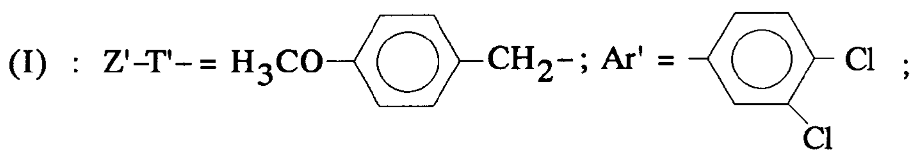

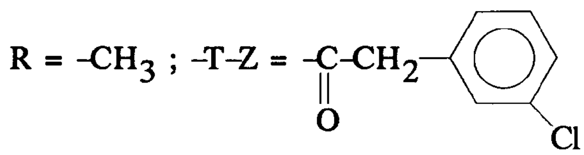

Ce composé est préparé selon la voie b du schéma 2.

A) 1-(3,4-Dichlorophényl)-3-[(1-trityl)-pipéridin-4-yl]-1-cyanopropane.

On dissout 25 g du chlorhydrate de 1-(3,4-dichlorophényl)-3-(4-pipéridinyl)-1-cyanopropane (exemple 1 B) dans 400 ml de $CH_2Cl_2$. On ajoute goutte à goutte 22 g de chlorure de trityle et 21 ml de triéthylamine à la solution. On laisse le mélange réactionnel pendant 2 heures sous agitation, lave successivement avec $H_2O$, une solution aqueuse tamponnée à pH = 2, une solution saturée de NACl. On sèche sur $MgSO_4$ et évapore. Le résidu est chromatographié sur gel de silice, éluant : heptane/acétate d'éthyle 9/1 (v/v). On obtient 39 g d'huile.

B) 2-(3,4-Dichlorophényl)-4-[(1-trityl)-pipéridin-4-yl]-butylamine.

L'huile préparée précédemment est dissoute dans 500 ml de glycolmonométhyléther ; on ajoute 50 ml d'ammoniaque et on procède à une hydrogénation à pression atmosphérique et température ambiante en présence de Ni de Raney. On évapore ensuite sous vide et on obtient 39 g d'huile.

C) N-methyl-4-[(1-trityl)-piperidin-4-yl]-2-(3,4-dichlorophenyl)-butylamine.

L'huile préparée précédemment est dissoute dans 250 ml de dichlorométhane à -15°C sous azote et traitée par 8 g de chloroformiate d'éthyle et 10 ml de triéthylamine. Après lavage avec NAOH 5 %, $H_2O$ et une solution saturée de NACl, on sèche la phase organique et évapore. L'huile obtenue est reprise dans 200 ml de THF et versée goutte à goutte sur 5,2 g de $LiAlH_4$ en suspension dans 200 ml de THF. Après 3 heures de reflux, on refroidit le mélange réactionnel et ajoute 27 ml d'$H_2O$. On filtre, évapore le filtrat et chromatographie le résidu sur gel de silice, éluant : $CH_2Cl_2$/MeOH 100/2 (v/v). On obtient 24 g d'huile.

D) N-methyl-N-[{4-[1-trityl-piperidin-4-yl]-2-(3,4-dichlorophenyl)}-butyl]-3-chlorophenylacétamide.

11,53 g de l'amine précédente, 3,8 g d'acide 3-chlorophénylacétique, 3 ml de triéthylamine, 9,73 g de BOP sont dissous successivement dans 200 ml de $CH_2Cl_2$. Après 30 minutes de réaction, on lave avec une solution de NAOH 5 %, $H_2O$, sèche sur $MgSO_4$ et évapore. Le résidu est chromatographié sur gel de silice, éluant : pentane/acétate d'éthyle 80/20 (v/v). On concentre les fractions pour obtenir 8 g d'une mousse.

E) Composé 23

8,0 g de l'amine obtenue précédemment sont dissous dans 50 ml d'acide formique ; on ajoute goutte à goutte 50 ml d'$H_2O$. On laisse réagir 30 minutes à 60°C, on filtre, évapore, reprend le résidu dans 100 ml d'$H_2O$, basifie avec une sodution de NAOH 30 % jusqu'à pH = 10, extrait avec 2 fois 150 ml d'éther éthylique, sèche

sur $MgSO_4$ et évapore. On obtient 6 g d'huile dont on prélève 2,2 g pour les dissoudre dans 15 ml de DMF. On ajoute 5 g de $K_2CO_3$ et 0,75 g de chlorure de paraméthoxybenzyle. On laisse réagir à 65°C pendant 2 heures puis on verse le mélange réactionnel dans 250 ml d'$H_2O$. On extrait deux fois avec 200 ml d'éther, lave avec $H_2O$, sèche la phase organique sur $MgSO_4$, filtre et évapore. Le résidu est purifié par chromatographie sur gel de silice, éluant : $CH_2Cl_2/CH_3OH$ 98/2 (v/v). La concentration des fractions pures fournit un résidu à partir duquel on prépare le chlorhydrate dans le dichlorométhane.

m =2 g

F = 100°C.

En procédant selon l'exemple 23, on prépare les composés décrits dans le Tableau 6 ci-dessous.

## TABLEAU 6

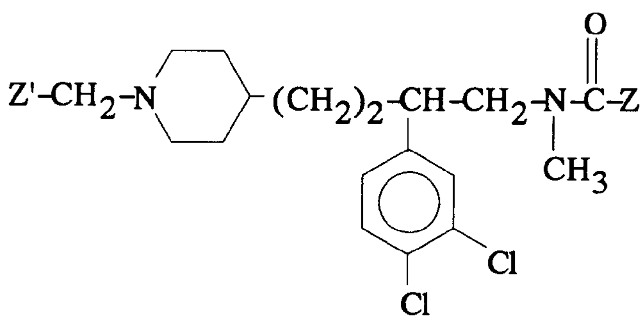

| Exemple n° | Z' | Z | F ; °C | Sel | Voie |
|---|---|---|---|---|---|
| 24 | 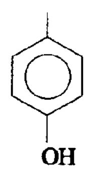 | 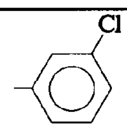 | 124 | HCl | b |
| 25 | 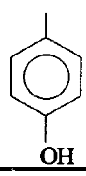 | 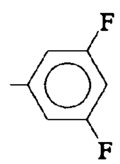 | 120 | HCl | b |

EXEMPLE 26

Chlorhydrate de N-méthyl-N-[{4-[1-hydroxybenzyl-piperidin-4-yl]-2-(3,4-dichlorophenyl)}-butyl]-3-isopropoxyphenylacétamide.

(I) : 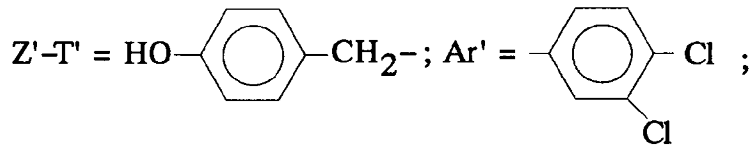

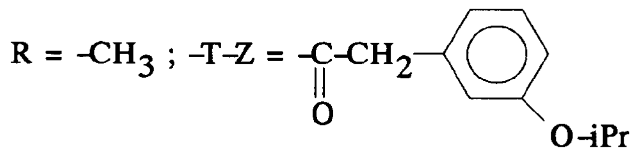

A) Ester méthylique de l'acide 4-(méthoxyméthyléther)benzoïque.

On opère selon : Synthesis, 1976, 244.

15,3 g de méthylparaben (4-hydroxyméthylbenzoate) sont dissous dans 500 ml de dichlorométhane. On ajoute 50 ml de diméthoxyméthane et une pointe de spatule d'acide paratoluènesulfonique. On laisse sous reflux au dessus d'un Soxhlet garni de tamis moléculaire de 3 Angström. Après 24 heures de réaction, on filtre le produit, lave successivement avec une solution de $NaHCO_3$, à l'eau et avec une solution saturée de NACl puis évapore la hase organique. On obtient 17 de liquide jaune.

B) 4-(Méthoxyméthylèther)benzylalcool.

A 5 g de $LiAlH_4$ en suspension dans 75 ml de THF, on ajoute 17 g du produit préparé précédemment dilué dans 100 ml de THF. On évapore et on chramatographie le résidu sur gel de silice, éluant : heptane/acétate d'éthyle 8/2 (v/v). On obtient 15 g d'huile incolore.

C) Chlorure de 4-(méthoxyméthyl)benzyléther.

10 g de l'huile précédente sont dissouts dans 20 ml d'acétonitrile puis, on ajoute, à 0°C, 15 g de triphénylphosphine et 8 g de N-chlorosuccinimide. Après 1 heure, on évapore, reprend le résidu dans l'éther, filtre, évapore le filtrat. Le résidu est chromatographié sur gel de silice, éluant : heptane/acétate d'éthyle 9/1 (v/v). On obtient 4,3 g de produit attendu.

D) N-méthyl-N-[{4-[1-trityl-piperidin-4-yl]-2-(3,4-dichlorophenyl)}-butyl]-3-isopropoxyphenylacétamide.

On dissout 3 g de l'amine préparée selon l'exemple 23 étape C, 1,05 g d'acide 3-isopropoxyphénylacétique,0,70 g de triéthylamine et 2,4 g de BOP dans 50 ml de $CH_2Cl_2$. On lave après 30 minutes de réaction avec une solution de NAOH à 5 %, à l'eau puis avec une solution de NACl saturée. On sèche sur $MgSO_4$ et évapore. On obtient 3,8 g d'huile.

E) N-méthyl-N-[{4-[1-(méthoxyméthyl)benzyléther-piperidin-4-yl]-2-(3,4-dichlorophényl)}butyl]-3-isopropoxyphénylacétamide.

L'huile obtenue précédemment est dissoute dans 35 ml d'acide formique ; on ajoute à la solution 25 ml d'$H_2O$ et laisse réagir 30 minutes à 60°C. On filtre, évapore le filtrat et redissout l'huile obtenue dans 50 ml d'$H_2O$. On basifie jusqu'à pH = 10, extrait à l'éther et sèche sur $MgSO_4$. On obtient 2,8 g d'huile après évaporation, qu'on dissout dans 20 ml de DMF. On ajoute 5 g de $K_2CO_3$, et 1,2 g de chlorure de 4-(méthoxyméthyl)benzyléther (préparé en C). Après 2 heures de réaction à 65°C, on verse dans 200 ml d'$H_2O$, extrait à l'éther, lave avec $H_2O$, sèche avec $MgSO_4$ et évapore. On obtient 3,5 g d'huile.

F) Composé 26

L'huile obtenue précédemment est diluée dans 25 ml de THF, 25 ml de 2-propanol et 15 ml de solution HCl/éther (4N). Après 2 heures sous agitation, on évapore et on chromatographie sur gel de silice, éluant: $CH_2Cl_2/CH_3OH$ 98/2 (v/v).

m = 2,5 g

F = 125°C.

EXEMPLE 27

Iodure de N-benzyl-1-methyl-4-{[3-(3,4-dichlorophenyl)-4-[N'-methyl-(3-isopropoxyphenyl)acetamido]]-butyl}-piperidinium.

(I) : 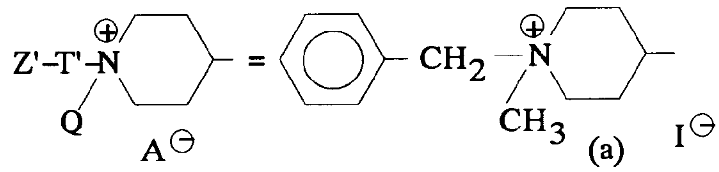 (a) ;

Ar' = 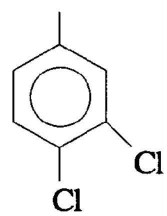 ; -T-Z = 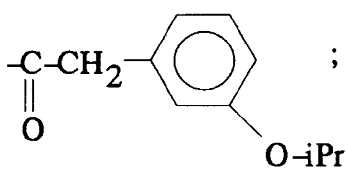 ; R = $-CH_3$

On dissout 1,5 g du composé préparé selon l'exemple 18 dans 50 ml de $CH_2Cl_2$. On agite le mélange avec 10 ml d'une solution de NAOH 10 %, sèche la phase organique sur $MgSO_4$, évapore. L'huile obtenue est dissoute dans 50 ml d'iodure de méthyle laissé pendant une heure à température ambiante puis évaporée. Le résidu est chromatographié sur gel de silice H, éluant : $CH_2Cl_2/CH_3OH$ 97/3 (v/v). Les fractions de produit pur sont concentrées.

m = 1,3 g

F = 108°C.

Le groupe méthyle en position 1 sur la pipéridine est de configuration axiale.

Spectre de RMN $^{13}C$:

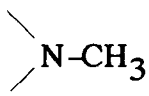 axial : δ = 44,18 ppm

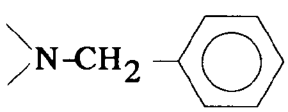 équatorial : δ = 69,76 ppm

EXEMPLE 28

Iodure de N-benzyl-1-methyl-4-{[3-(3,4-dichlorophenyl)-4-[N'-methyl-(3-isopropoxyphenyl)acetamido]]-butyl}-piperidinium.

(I) : 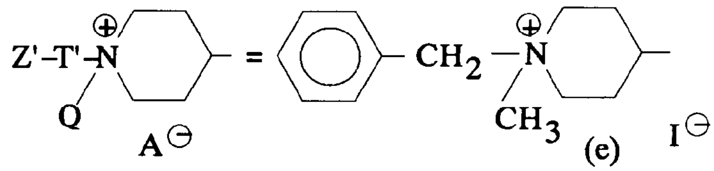 ;

Ar' = 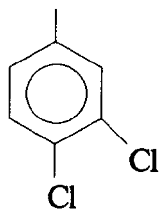 ; -T-Z = 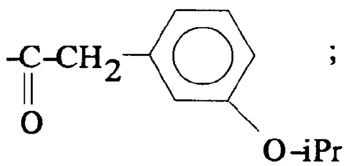 ; R = $-CH_3$

On élue la colonne de chromatographie précédente avec un mélange $CH_2Cl_2/CH_3OH$ 95/5 (v/v) pour obtenir 0,20 g d'une fraction correspondant au produit dont le méthyle en position 1 de la pipéridine est de configuration équatoriale.

F = 105°C.

Spectre de RMN $^{13}C$:

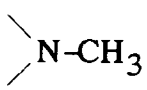 équatorial : δ = 51,20 ppm

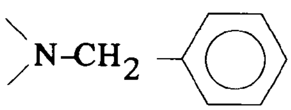 axial : δ = 59,85 ppm

**Revendications**

**1** Dérivés aromatiques aminés de formule :

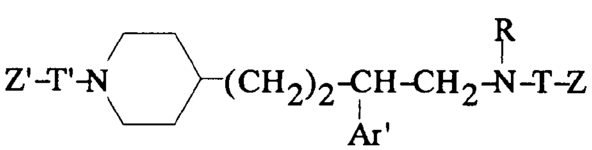

(I)

dans laquelle :

- Ar' représente un phényle non substitué ou substitué une ou plusieurs fois par un atome d'halogène, de préférence un atome de chlore ou de fluor, par un alkyle en $C_1$-$C_3$, par un trifluorométhyle, par un alcoxy en $C_1$-$C_3$, par un hydroxyle ; un groupe thiényle, pyridyle, naphtyle, lesdits groupes étant non substitués

ou substitués par un halogène de préférence un atome de chlore ou de fluor, un groupe indolyle ou un groupe benzothiényle ;

- R représente l'hydrogène, un groupe méthyle ou un groupe $(CH_2)_n$-L, où n est un nombre entier de 2 à 6 et L est l'hydrogène ou un groupe amino ;
- Z et Z représentent indépendamment un atome d'hydrogène ou un groupe M ou OM,

M représente l'hydrogène, un alkyle droit ou ramifié en $C_1$-$C_6$ ; un α-hydroxybenzyle, un α-alkylbenzyle ou un phénylalkyle dans lequel le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone ; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (1-méthyl) 2-imidazolylthioalkyle dans lequel le groupe alkyle comprend de 1 à 3 atomes de carbone ; un 1-oxo-3-phénylindan-2-yle ; un groupe aromatique ou hétéroaromatique ledit groupe étant non substitué ou substitué ;

- T' représente une liaison, un groupe $-CH_2-$ ou, un groupe -C(O)-;
- T représente un groupe choisi parmi

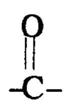

et

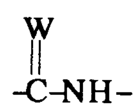

W étant un atome d'oxygène ou de soufre, avec la limitation que

. lorsque Z' est hydrogène ou OM, T' est autre qu'une liaison; et

. lorsque Z est hydrogène ou OM, T est autre qu'un groupe -C(W)-NH;

ou un de leurs sels éventuels avec des acides minéraux ou organiques ou un de leurs sels d'ammonium quaternaire.

**2** Composés selon la revendication 1, caractérisés en ce qu'ils sont sous la forme d'un sel d'ammonium quaternaire, le groupe de formule

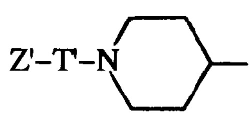

étant alors représenté par la formule:

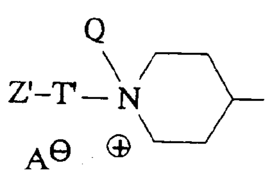

dans laquelle

. Q représente un groupe alkyle en $C_1$-$C_6$ ou un groupe benzyle et

. $A^{\ominus}$ représente un anion choisi parmi chlorure, bromure, iodure, acétate, méthanesulfonate ou paratoluènesulfonate.

**3** Composés selon la revendication 1 caractérisés en ce que Z représente un groupe phényle ou un groupe naphtyle, lesdits groupes étant non substitués ou substitués par un halogène, par un alkyle ou par un alcoxy ou un de leurs sels pharmaceutiquement acceptables.

**4** Composés selon la revendication 1 caractérisés en ce que Ar' représente un groupe phényle non substitués ou substitués une ou plusieurs fois par un halogène, ou un de leurs sels pharmaceutiquement acceptables.

**5** Composés selon la revendication 1 caractérisés en ce que T represente un groupe

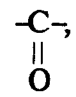

ou un de leurs sels pharmaceutiquement acceptables.

**6** Procédé pour la préparation des composés de formule (I) selon la revendication 1 caractérisé en ce qu'un nitrile de formule :

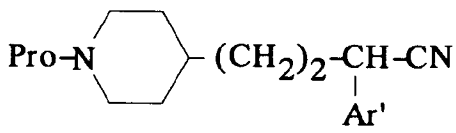

(III)

dans lequel Ar' est tel que défini dans la revendication 1, et Pro désigne un groupe N-protecteur, est :
- soit soumis à une hydrogénation catalytique en présence d'un catalyseur tel que par exemple le nickel de Raney pour fournir l'amine de formule :

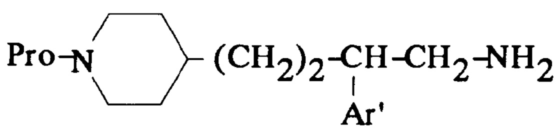

(IV)

laquelle, après introduction éventuelle du radical R différent de l'hydrogène, est ensuite fonctionnalisée

. soit avec un dérivé fonctionnel d'un acide de formule

HO - CO - Z (V)

dans laquelle Z est tel que défini dans la revendication 1, lorsqu'on doit préparer un composé de formule (I) dans lequel T est - CO-,

. soit avec iso(thio)cyanate de formule

W = C = N - Z (VI)

dans laquelle W et Z sont tels que définis dans la revendication 1 lorsqu'on doit préparer un composé de formule (I) dans lequel T est -C (W) - NH -, pour fournir le dérivé

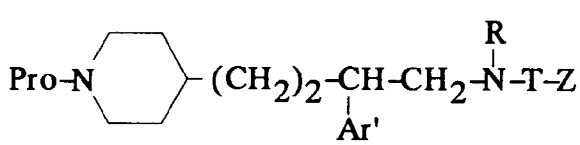

(VII)

qui est alors déprotégé pour conduire à l'amine libre de formule :

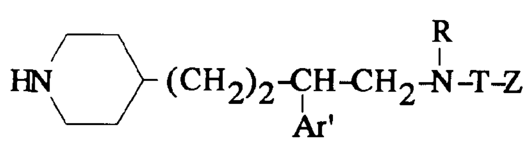

(VIII)

dans laquelle Ar', T, R et Z sont tels que définis dans la revendication 1, puis cette amine est additionnée sur un dérivé halogéné de formule

Z′ - T′ - Hal (IX)

dans laquelle Z′ et T′ sont tels que définis dans la revendication 1 et où Hal représente un halogène, plus particulièrement un atome de chlore et de brome, pour former les composés (I);

- soit l'aminonitrile (III) est déprotégé pour conduire à l'aminonitrile libre de formule:

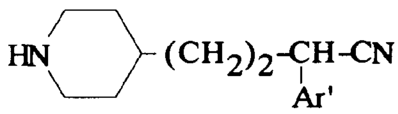

(X)

dans laquelle Ar′ est tel que défini dans la revendication 1, et sur lequel on additionne le dérivé halogéné (IX) pour conduire au nitrile de formule :

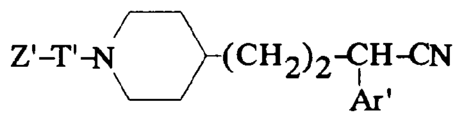

(XI)

qui est ensuite hydrogéné en présence d'un catalyseur tel que le nickel de Raney pour fournir le dérivé aminé correspondant de formule :

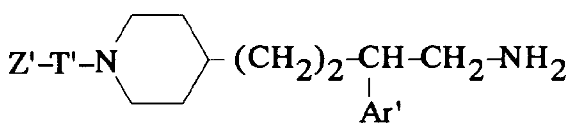

(XII)

qui, après introduction éventuelle du radical R différent de l'hydrogène, est ensuite fonctionnalisé pour fournir le composé (I) en le faisant réagir:

. soit avec un dérivé fonctionnel d'un acide de formule

HO - CO - Z (V)

dans laquelle Z est tel que défini dans la revendication 1, lorsqu'on doit préparer un composé de formule (I) dans lequel T est - CO -,

. soit avec un iso(thio)cyanate de formule

W = C = N = Z (VI)

dans laquelle W et Z sont tels que définis dans la revendication 1 pour préparer un composé de formule (I) dans lequel T est - C (W) - NH -;

- soit l'aminonitrile (III) est soumis à une hydrogénation catalytique en présence d'un catalyseur tel que par exemple le nickel de Raney pour fournir le composé de formule (IV) qui, après éventuelle introduction du radical R différent de l'hydrogène et déprotection, donne l'amine libre de formule (IV') qui est additionnée sur un dérivé halogéné de formule

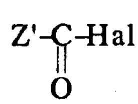

dans laquelle Z′ est tel que défini dans la revendication 1,

pour fournir les dérivés de formule (I) lesquels sont ensuite éventuellement transformés en leurs sels avec des acides minéraux ou organiques ou en leurs sels d'ammonium quaternaire.

**7** Composé de formule :

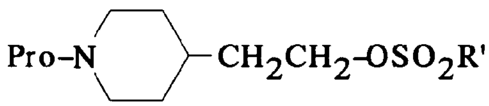

(II)

dans laquelle
- Pro est un groupe N-protecteur,
- R′ représente un groupe alkyle en $C_1$-$C_4$, un groupe phényle ou un groupe tolyle, de préférence paratolyle,
ou un de ses sels.

**8** Composé de formule :

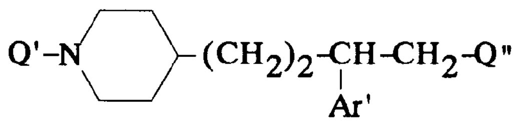

(XXII)

dans laquelle
Ar′ est tel que défini dans la revendication 1, et
- soit Q′ est l'hydrogène ou un groupe N-protecteur et Q″ est un groupe amino ou un groupe :

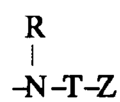

où R, T et Z sont tels que définis dans la revendication 1,
- soit Q′ est un groupe :

Z′-T′

où Z′ et T′ sont tels que définis dans la revendication 1 et Q″ est un groupe amino, et ses sels d'addition acides.

**9** Composé de formule :

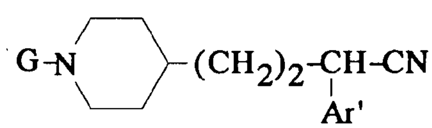

(XXIII)

dans laquelle
- G est l'hydrogène, un groupe protecteur (Pro) ou un groupe de formule

Z′-T′

dans lequel Z′ et T′ sont tels que définis dans la revendication 1, et
- Ar′ est tel que défini dans la revendication 1, ou un de ses sels.

**10** Composition pharmaceutique contenant, en tant que principe actif, un composé de formule (I) selon la revendication 1.

**11** Composition pharmaceutique selon la revendication 10, sous forme d'unité de dosage, dans laquelle le principe actif est mélangé à au moins un excipient pharmaceutique.

**12** Composition selon la revendication 11 contenant de 2,5 à 1000 mg de principe actif.

**Revendications pour l'Etat contractant suivant : ES**

**1** Procédé pour l'obtention de dérivés aromatiques aminés de formule :

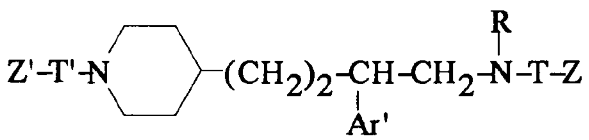

(I)

dans laquelle :

- Ar′ représente un phényle non substitué ou substitué une ou plusieurs fois par un atome d'halogène, de préférence un atome de chlore ou de fluor, par un alkyle en $C_1$-$C_3$, par un trifluorométhyle, par un alcoxy en $C_1$-$C_3$, par un hydroxyle ; un groupe thiényle, pyridyle, naphtyle, lesdits groupes étant non substitués ou substitués par un halogène de préférence un atome de chlore ou de fluor, un groupe indolyle ou un groupe benzothiényle ;
- R représente l'hydrogène, un groupe méthyle ou un groupe $(CH_2)_n$-L, où n est un nombre entier de 2 à 6 et L est l'hydrogène ou un groupe amino ;
- Z et Z′ représentent indépendamment un atome d'hydrogène ou un groupe M ou OM,

M représente l'hydrogène, un alkyle droit ou ramifié en $C_1$-$C_6$ ; un $\alpha$-hydroxybenzyle, un $\alpha$-alkylbenzyle ou un phénylalkyle dans lequel le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone ; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (1-méthyl) 2-imidazolylthioalkyle dans lequel le groupe alkyle comprend de 1 à 3 atomes de carbone ; un 1-oxo-3-phénylindan-2-yle ; un groupe aromatique ou hétéroaromatique ledit groupe étant non substitué ou substitué ;

- T′ représente une liaison, un groupe $-CH_2-$ ou, un groupe -C(O)-;
- T représente un groupe choisi parmi

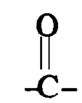

et

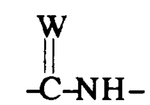

W étant un atome d'oxygène ou de soufre, avec la limitation que

. lorsque Z′ est hydrogène ou OM, T′ est autre qu'une liaison; et

. lorsque Z est hydrogène ou OM, T est autre qu'un groupe -C(W)-NH;

ou un de leurs sels éventuels avec des acides minéraux ou organiques ou un de leurs sels d'ammonium quaternaire, caractérisé en ce qu'un nitrile de formule :

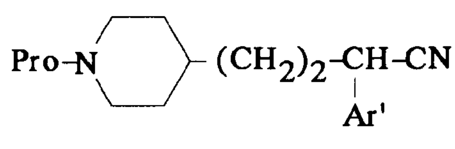

(III)

dans lequel Ar′ est tel que défini précédemment, et Pro désigne un groupe N-protecteur, est :

- soit soumis à une hydrogénation catalytique en présence d'un catalyseur tel que par exemple le nickel de Raney pour fournir l'amine de formule :

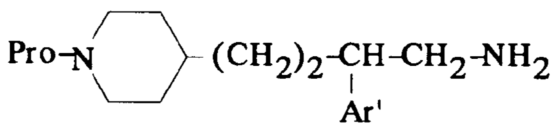

(IV)

laquelle, après introduction éventuelle du radical R différent de l'hydrogène, est ensuite fonctionnalisée

. soit avec un dérivé fonctionnel d'un acide de formule

HO - CO - Z (V)

dans laquelle Z est tel que défini précédemment, lorsqu'on doit préparer un composé de formule (I) dans lequel T est - CO-,

. soit avec iso(thio)cyanate de formule

W = C = N - Z (VI)

dans laquelle W et Z sont tels que définis précédemment lorsqu'on doit préparer un composé de formule (I) dans lequel T est -C (W) - NH -, pour fournir le dérivé

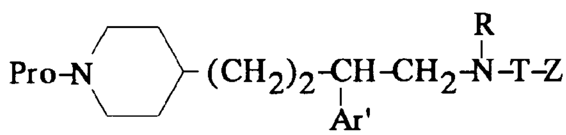

(VII)

qui est alors déprotégé pour conduire à l'amine libre de formule :

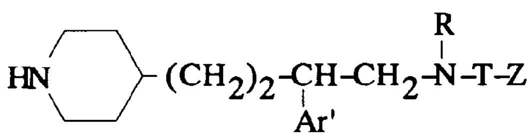

(VIII)

dans laquelle Ar', T, R et Z sont tels que définis précédemment, puis cette amine est additionnée sur un dérivé halogéné de formule

Z' - T' - Hal (IX)

dans laquelle Z' et T sont tels que définis précédemment et où Hal représente un halogène, plus particulièrement un atome de chlore et de brome, pour former les composés (I);

- soit l'aminonitrile (III) est déprotégé pour conduire à l'aminonitrile libre de formule:

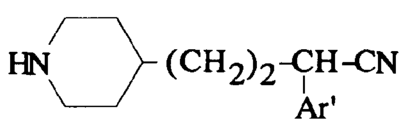

(X)

dans laquelle Ar' est tel que défini précédemment, et sur lequel on additionne le dérivé halogéné (IX) pour conduire au nitrile de formule :

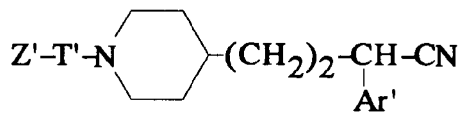

(XI)

qui est ensuite hydrogéné en présence d'un catalyseur tel que le nickel de Raney pour fournir le dérivé aminé correspondant de formule :

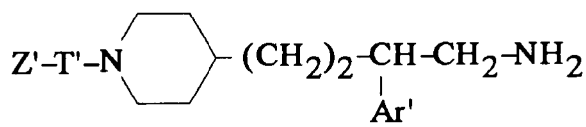

(XII)

qui, après introduction éventuelle du radical R différent de l'hydrogène, est ensuite fonctionnalisé pour fournir le composé (I) en le faisant réagir:

. soit avec un dérivé fonctionnel d'un acide de formule

HO - CO - Z (V)

dans laquelle Z est tel que défini précédemment, lorsqu'on doit préparer un composé de formule (I) dans lequel T est - CO -,

. soit avec un iso(thio)cyanate de formule

W = C = N = Z (VI)

dans laquelle W et Z sont tels que définis précédemment pour préparer un composé de formule (I) dans lequel T est - C (W) - NH -;

- soit l'aminonitrile (III) est soumis à une hydrogénation catalytique en présence d'un catalyseur tel que par exemple le nickel de Raney pour fournir le composé de formule (IV) qui, après éventuelle introduction du radical R différent de l'hydrogène et déprotection, donne l'amine libre de formule (IV') qui est additionnée sur un dérivé halogéné de formule

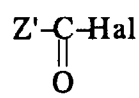

dans laquelle Z′ est tel que défini précédemment,

pour fournir les dérivés de formule (I) lesquels sont ensuite éventuellement transformés en leurs sels avec des acides minéraux ou organiques ou en leurs sels d'ammonium quaternaire.

**2** Procédé selon la revendication 1, caractérisé en ce que l'étape de transformation des composés de formule (I) en leurs sels d'ammonium quaternaire consiste à faire réagir les bases libres des composés de formule (I), pour lesquelles lesdites fonctions aminées autres, éventuellement présentes, sont N-protégées par un groupe N-protecteur, avec un excès d'un agent alkylant de formule :

A - Q

dans lequel A représente un groupe partant et est tel que défini ci-après et Q′ est tel que défini dans la revendication 1 pour les composés de formule (I) et à chauffer le mélange réactionnel dans un solvant choisi par exemple parmi le dichlorométhane, le chloroforme, l'acétone ou l'acétonitrile à une température comprise entre la température ambiante et le reflux du solvant pendant une à plusieurs heures pour obtenir, après traitement habituel et déprotection éventuelle, un mélange de conformères axiaux et équatoriaux des sels d'ammonium quaternaire des composés de formule (I) dans laquelle le groupe

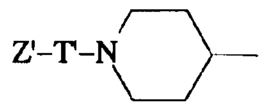

est représenté par le groupe de formule :

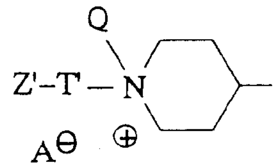

dans laquelle

. Q représente un groupe alkyle en $C_1$-$C_6$ ou un groupe benzyle et

. $A^{\ominus}$ représente un anion choisi parmi chlorure, bromure, iodure, acétate, méthanesulfonate ou paratoluènesulfonate.

**3** Procédé selon la revendication 1, caractérisé en ce que l'on utilise un composé de formule (V) ou (VI), dans lesquelles Z représente un groupe phényle ou un groupe naphtyle, lesdits groupes étant non substitués ou substitués par un halogène, par un alkyle ou par un alcoxy.

**4** Procédé selon l'une des revendications 1 ou 3, caractérisé en ce que l'on utilise un composé de formule (III) ou un composé de formule (X) dans lesquels Ar' représente un groupe phényle non substitué ou substitué une ou plusieurs fois par un halogène.

**5** Procédé selon l'une quelconque des revendications 1 ou 4, caractérisé en ce qu'on utilise un composé de formule (I) dans laquelle T représente le groupe

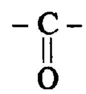

**6** Procédé pour l'obtention d'un composé de formule :

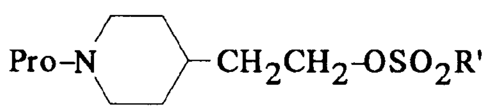

(II)

dans laquelle

- Pro est un groupe N-protecteur,
- R' représente un groupe alkyle en $C_1$-$C_4$, un groupe phényle ou un groupe tolyle, de préférence paratolyle,

ou un de ses sels, caractérisé en ce qu'il consiste à faire réagir le 1-hydroxy-2(4-pipéridinyl)éthane avec un agent de N-protection pour former le dérivé N-protégé correspondant, puis à faire réagir ledit dérivé avec $R'SO_2$ Cl pour former le composé de formule (II) et à transformer éventuellement le composé ainsi obtenu en l'un de ses sels.

**7** Procédé pour l'obtention d'un composé de formule :

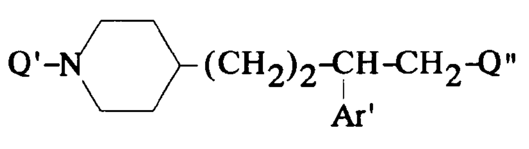

(XXII)

dans laquelle

Ar′ est tel que défini dans la revendication 1, et

- soit Q′ est l'hydrogène ou un groupe N-protecteur et Q″ est un groupe amino ou un groupe :

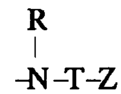

où R, T et Z sont tels que définis dans la revendication 1,

- soit Q′ est un groupe :

Z′-T′

où Z′ et T′ sont tels que définis dans la revendication 1 et Q″ est un groupe amino,

et ses sels d'addition acides, caractérisé en ce qu'il consiste :

1) à faire réagir le composé de formule :

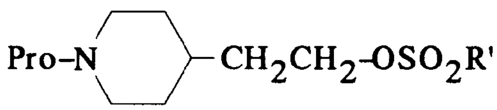

(II)

dans laquelle

- Pro est un groupe N-protecteur,
- R′ représente un groupe alkyle en $C_1$-$C_4$, un groupe phényle ou un groupe tolyle, de préférence para-tolyle, avec un nitrile de formule : Ar′ - $CH_2$ - CN dans laquelle Ar′ est tel que défini ci-dessus pour former l'aminonitrile de formule (III)

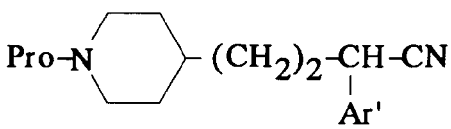

(III)

dans laquelle Pro et Ar′ sont tels que définis précédemment et à soumettre ledit nitrile (III) à un hydrogénation catalytique en présence d'un catalyseur, tel que par exemple le nickel de Raney, pour former le composé de formule (XXII) dans laquelle Q′ est Pro et Q″ est un groupe amino ;

2) éventuellement à faire réagir le composé de formule (XXII) ainsi obtenu :

. soit avec un dérivé fonctionnel d'un acide de formule

HO - CO - Z (V)

dans laquelle Z est tel que défini précédemment, lorsqu'on doit préparer un composé de formule (I) dans lequel T est - CO-,

. soit avec iso(thio)cyanate de formule

W = C = N - Z (VI)

dans laquelle W et Z sont tels que définis précédemment pour fournir le dérivé de formule (XXII) dans laquelle Q′ est Pro et Q″ représente le groupe

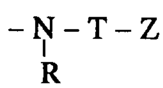

dans lequel R, T et Z sont tels que définis dans la revendication 1;

3) éventuellement à déprotéger l'aminonitrile de formule (III) pour former l'aminonitrile libre de formule :

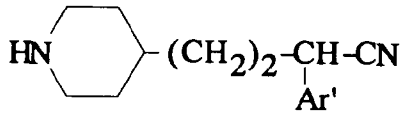

(X)

dans laquelle Ar′ est tel que défini précédemment, et sur lequel on additionne le dérivé halogéné (IX) pour conduire au nitrile de formule :

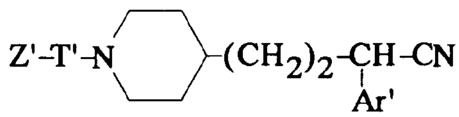

(XI)

qui est ensuite hydrogéné en présence d'un catalyseur tel que le nickel de Raney pour fournir le dérivé aminé correspondant de formule (XXII) dans laquelle Q′ représente Z′ - T -, Z′ et T étant tels que définis dans la revendication 1 et Q″ est un groupe amino et

4) éventuellement à transformer lesdits composés ainsi obtenus en 1), 2) ou 3) en leurs sels.

**8** Procédé pour l'obtention d'un composé de formule :

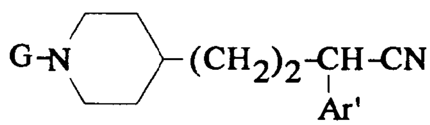

(XXIII)

dans laquelle

- G est l'hydrogène, un groupe protecteur ou un groupe de formule

Z′-T′

dans lequel Z′ et T′ sont tels que définis dans la revendication 1, et

- Ar′ est tel que défini dans la revendication 1 ou un de ses sels, caractérisé en ce qu'il consiste :

1) à faire réagir le composé de formule :

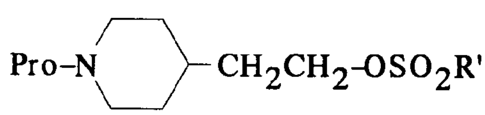

(II)

dans laquelle

- Pro est un groupe N-protecteur,
- R′ représente un groupe alkyle en $C_1$-$C_4$, un groupe phényle ou un groupe tolyle, de préférence para-tolyle, avec un nitrile de formule : Ar′ - $CH_2$ - CN dans laquelle Ar′ est tel que défini ci-dessus pour former le composé de formule (XXIII) dans laquelle G est un groupe protecteur ;

2) éventuellement à déprotéger ledit composé ainsi obtenu pour former le composé de formule (XXIII) dans laquelle G est l'hydrogène ;

3) éventuellement à faire réagir ledit composé ainsi obtenu sur un dérivé halogéné de formule :

Z′ - T′ - Hal (IX)

dans laquelle Z′ et T′ sont tels que définis dans la revendication 1, pour former les composés de formule (XXIII) dans laquelle G est le groupe Z′- T′ et à transformer éventuellement lesdits composés

ainsi obtenus en 1 , 2 ou 3 en leurs sels.

**9** Procédé pour la préparation d'une composition pharmaceutique, caractérisé en ce que l'on mélange, en tant que principe actif, un composé de formule (I) préparé par le procédé selon l'une quelconque des revendications 1 à 5 avec un véhicule pharmaceutiquement acceptable.

**Revendications pour l'Etat contractant suivant : GR**

**1** Dérivés aromatiques aminés de formule :

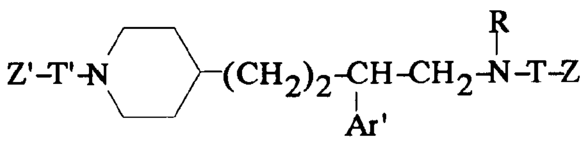

(I)

dans laquelle :

- Ar' représente un phényle non substitué ou substitué une ou plusieurs fois par un atome d'halogène, de préférence un atome de chlore ou de fluor, par un alkyle en $C_1$-$C_3$, par un trifluorométhyle, par un alcoxy en $C_1$-$C_3$, par un hydroxyle ; un groupe thiényle, pyridyle, naphtyle, lesdits groupes étant non substitués ou substitués par un halogène de préférence un atome de chlore ou de fluor, un groupe indolyle ou un groupe benzothiényle ;
- R représente l'hydrogène, un groupe méthyle ou un groupe $(CH_2)_n$-L, où n est un nombre entier de 2 à 6 et L est l'hydrogène ou un groupe amino;
- Z et Z' représentent indépendamment un atome d'hydrogène ou un groupe M ou OM,

M représente l'hydrogène, un alkyle droit ou ramifié en $C_1$-$C_6$ ; un $\alpha$-hydroxybenzyle, un $\alpha$-alkylbenzyle ou un phénylalkyle dans lequel le groupe alkyle comprend de 1 à 3 atomes de carbone, non substitué, mono ou polysubstitué sur le cycle aromatique par un halogène, un hydroxy, un alcoxy de 1 à 4 atomes de carbone, un alkyle de 1 à 4 atomes de carbone ; un pyridylalkyle dans lequel le groupe alkyle comprend de 1 à 3 atomes de carbone ; un naphtylalkyle dans lequel le groupe alkyle comprend de 1 à 3 atomes de carbone ; un pyridylthioalkyle dans lequel le groupe alkyle comprend de 1 à 3 atomes de carbone ; un styryle ; un (1-méthyl) 2-imidazolylthioalkyle dans lequel le groupe alkyle comprend de 1 à 3 atomes de carbone ; un 1-oxo-3-phénylindan-2-yle ; un groupe aromatique ou hétéroaromatique ledit groupe étant non substitué ou substitué ;

- T' représente une liaison, un groupe $-CH_2-$ ou, un groupe -C(O)-;
- T représente un groupe choisi parmi

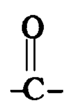

et

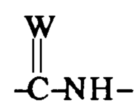

W étant un atome d'oxygène ou de soufre, avec la limitation que

. lorsque Z' est hydrogène ou OM, T' est autre qu'une liaison; et

. lorsque Z est hydrogène ou OM, T est autre qu'un groupe -C(W)-NH ;

ou un de leurs sels éventuels avec des acides minéraux ou organiques ou un de leurs sels d'ammonium quaternaire.

**2** Composés selon la revendication 1, caractérisés en ce qu'ils sont sous la forme d'un sel d'ammonium quaternaire, le groupe de formule

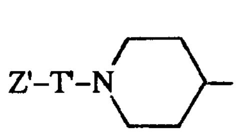

étant alors représenté par la formule:

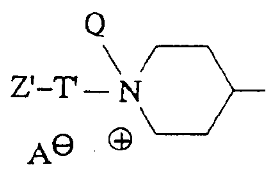

dans laquelle

. Q représente un groupe alkyle en $C_1$-$C_6$ ou un groupe benzyle et

. A $^{\circ}$ représente un anion choisi parmi chlorure, bromure, iodure, acétate, méthanesulfonate ou paratoluènesulfonate.

**3** Composés selon la revendication 1 caractérisés en ce que Z représente un groupe phényle ou un groupe naphtyle, lesdits groupes étant non substitués ou substitués par un halogène, par un alkyle ou par un alcoxy ou un de leurs sels pharmaccutiquement acceptables.

**4** Composés selon la revendication 1 caractérisés en ce que Ar′ représente un groupe phényle non substitués ou substitués une ou plusieurs fois par un halogène, ou un de leurs sels pharmaceutiquement acceptables.

**5** Composés selon la revendication 1 caractérisés en ce que T représente un groupe

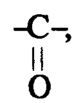

ou un de leurs sels pharmaceutiquement acceptables.

**6** Procédé pour la préparation des composés de formule (I) selon la revendication 1 caractérisé en ce qu'un nitrile de formule :

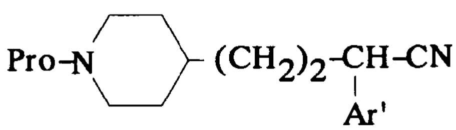

(III)

dans lequel Ar′ est tel que défini dans la revendication 1, et Pro désigne un groupe N-protecteur, est :

- soit soumis à une hydrogénation catalytique en présence d'un catalyseur tel que par exemple le nickel de Raney pour fournir l'amine de formule :

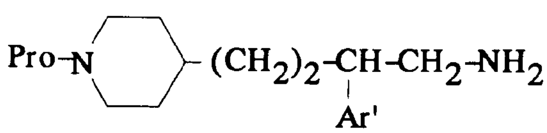

(IV)

laquelle, après introduction éventuelle du radical R différent de l'hydrogène, est ensuite fonctionnalisée

. soit avec un dérivé fonctionnel d'un acide de formule

HO - CO - Z (V)

dans laquelle Z est tel que défini dans la revendication 1, lorsqu'on doit préparer un composé de formule (I) dans lequel T est - CO-,

. soit avec iso(thio)cyanate de formule

W = C = N - Z (VI)

dans laquelle W et Z sont tels que définis dans la revendication 1 lorsqu'on doit préparer un composé de formule (I) dans lequel T est -C (W) - NH -, pour fournir le dérivé

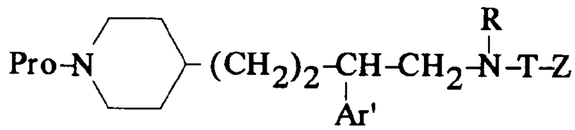

(VII)

qui est alors déprotégé pour conduire à l'amine libre de formule :

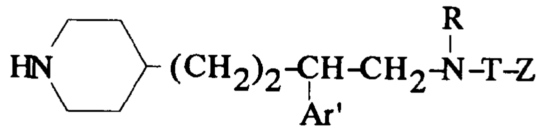

(VIII)

dans laquelle Ar', T, R et Z sont tels que définis dans la revendication 1, puis cette amine est additionnée sur un dérivé halogéné de formule

Z' - T' - Hal (IX)

dans laquelle Z' et T' sont tels que définis dans la revendication 1 et où Hal représente un halogène, plus particulièrement un atome de chlore et de brome, pour former les composés (I);

- soit l'aminonitrile (III) est déprotégé pour conduire à l'aminonitrile libre de formule:

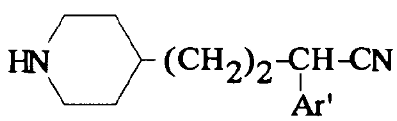

(X)

dans laquelle Ar' est tel que défini dans la revendication 1, et sur lequel on additionne le dérivé halogéné (IX) pour conduire au nitrile de formule :

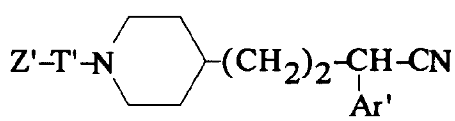

(XI)

qui est ensuite hydrogéné en présence d'un catalyseur tel que le nickel de Raney pour fournir le dérivé aminé correspondant de formule :

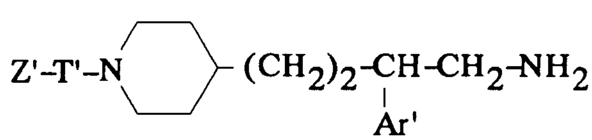

(XII)

qui, après introduction éventuelle du radical R différent de l'hydrogène, est ensuite fonctionnalisé pour fournir le composé (I) en le faisant réagir:

. soit avec un dérivé fonctionnel d'un acide de formule

HO - CO - Z (V)

dans laquelle Z est tel que défini dans la revendication 1, lorsqu'on doit préparer un composé de formule (1) dans lequel T est - CO -,

. soit avec un iso(thio)cyanate de formule

W = C = N = Z (VI)

dans laquelle W et Z sont tels que définis dans la revendication 1 pour préparer un composé de formule (I) dans lequel T est - C (W) - NH -;

- soit l'aminonitrile (III) est soumis à une hydrogénation catalytique en présence d'un catalyseur tel que par exemple le nickel de Raney pour fournir le composé de formule (IV) qui, après éventuelle introduction du radical R différent de l'hydrogène et déprotection, donne l'amine libre de formule (IV') qui est additionnée sur un dérivé halogéné de formule

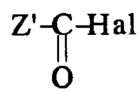

dans laquelle Z est tel que défini dans la revendication 1,

pour fournir les dérivés de formule (I) lesquels sont ensuite éventuellement transformés en leurs sels avec des acides minéraux ou organiques ou en leurs sels d'ammonium quaternaire.

**7** Composé de formule :

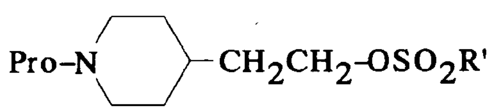

(II)

dans laquelle

- Pro est un groupe N-protecteur,
- R' représente un groupe alkyle en $C_1$-$C_4$, un groupe phényle ou un groupe tolyle, de préférence paratolyle,

ou un de ses sels.

**8** Composé de formule :

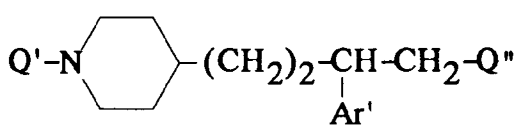

(XXII)

dans laquelle

Ar' est tel que défini dans la revendication 1, et

- soit Q' est l'hydrogène ou un groupe N-protecteur et Q" est un groupe amino ou un groupe :

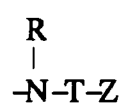

où R, T et Z sont tels que définis dans la revendication 1,

- soit Q' est un groupe :

Z'-T'

où Z' et T' sont tels que définis dans la revendication 1 et Q" est un groupe amino, et ses sels d'addition acides.

**9** Composé de formule :

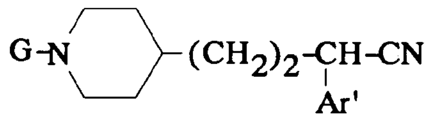

(XXIII)

dans laquelle

- G est l'hydrogène, un groupe protecteur (Pro) ou un groupe de formule

Z′-T′

dans lequel Z′ et T′ sont tels que définis dans la revendication 1, et

- Ar′ est tel que défini dans la revendication 1 ou un de ses sels.

**10** Procédé pour la préparation d'une composition pharmaceutique, caractérisé en ce que l'on mélange, en tant que principe actif, un composé de formule (I) selon l'une quelconque des revendications 1 à 5, avec un véhicule pharmaceutiquement acceptable.

Office européen des brevets

**RAPPORT PARTIEL DE RECHERCHE EUROPEENNE**
qui selon la règle 45 de la Convention sur le brevet européen est consideré, aux fins de la procédure ultérieure comme le rapport de la recherche européenne

Numero de la demande: EP 92 40 1236

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 330 026 (TAKEDA CHEMICAL IND.,LTD)<br>--- | | C 07 D 211/26<br>C 07 D 401/06<br>A 61 K 31/445 |
| A | US-A-4 604 393 (PIERRE-JEAN CORNU, et al.)<br>--- | | |
| A | FR-A-1 211 691 (MAY AND BAKER LTD)<br>--- | | |
| A | US-A-4 310 532 (THE UNIVERSITY OF TOLEDO)<br>--- | | |
| A | FR-A-2 528 835 (BOUCHARA EMILE)<br>----- | | |

DOMAINES TECHNIQUES RECHERCHES (Int. Cl.5)

C 07 D
A 61 K

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches complètes: 7
Revendications ayant fait l'objet de recherches incomplètes: 1-6,8-12
Revendications n'ayant pas fait l'objet de recherches:
Raison pour la limitation de la recherche:

Voir annex -C-

| Lieu de la recherche | Date d'achèvement de la recherche | Examinateur |
|---|---|---|
| LA HAYE | 31-07-1992 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0408)

EP 92 40 1236 -C-

La rédaction des revendications n'est pas claire ni concise (Art.83-84 OEB), et représente une telle masse énorme de produits, qu'une recherche complète n'est pas possible pour des raisons d'économie (voir Directives relatives à l'examen pratiqué à l'OEB, Partie B, Chapitre III,2). De cette façon la recherche a été fondé sur le concept inventif de la demande telle qu'exemplifié par les composés, qui sont bien caractérisés par des éléments physiques ou chimiques, c.a.d. les composés des exemples,